# EUROPEAN PATENT SPECIFICATION

(11) **EP 4 519 233 B1**
(45) Date of publication and mention of the grant of the patent: **04.02.2026**
(21) Application number: 23723636.9
(22) Date of filing: 03.05.2023
(51) Int. Cl.: C07C 29/149, C07C 33/025, C07C 33/03, C07C 33/02

(54) **PROCESS FOR THE HYDROGENATION OF UNSATURATED ESTERS**
VERFAHREN ZUR HYDRIERUNG VON UNGESÄTTIGTEN ESTERN
PROCÉDÉ D'HYDROGÉNATION D'ESTERS INSATURÉS

(30) Priority: 03.05.2022 GB 202206460
(43) Date of publication of application: 12.03.2025
(73) Proprietor: Johnson Matthey Public Limited Company, London EC2V 7AD (GB)
(72) Inventor: GRAINGER, Damian, Cambridge CB4 0FP (GB); VAJARIA, Krisha, Cambridge CB4 0FP (GB)
(74) Representative: Johnson Matthey Plc
(86) International application number: PCT/GB2023/051169
(87) International publication number: WO 2023/214163

(56) References cited:
- WO-A1-2014/139030
- WO-A1-2019/138216

## Description

### Field of the Invention

The present invention relates to a process for the hydrogenation of ester-containing substrates. More specifically, the present invention relates to a process for the reduction of α,β-γ,δ unsaturated containing esters and β,γ unsaturated containing esters.

### Background of the Invention

The reduction of esters is an essential transformation in the chemical industry as a route to primary alcohols. The reduction of esters has conventionally been carried out using reagents (in stoichiometric or excess quantities) such as sodium metal in ethanol (the Bouveault-Blanc reduction) or more recently with a metal hydride reagent, such as LiAlH₄ or NaBH₄. These reduction reactions are, however, difficult to carry out effectively on a large scale, not least due to safety concerns associated with an extremely exothermic quenching step. As such, research into the reduction of esters has more recently focussed on catalytic reduction using hydrogen gas. For example, Cu- or Zn-based heterogeneous catalysts are used for ester reduction, primarily in the Natural Detergent Alcohol (NDA) market on very large scale. However, these methods require very high pressures and/or temperatures, in addition to large scale, dedicated production facilities. The chemoselectivity for ester reduction compared to other sensitive functional groups can also be problematic in some cases using these methods.

The reduction of unsaturated esters, such as α,β-γ,δ unsaturated esters and β,γ unsaturated esters, can be particularly problematic. Finding conditions which are chemoselective for the reduction of the ester group whilst maintaining the regiochemistry of the alkenyl functional group(s) is particularly challenging.

As will be readily understood, α,β,γ, and δ, when used in the context of unsaturated esters, refer to the carbon atoms in an ester-containing substrate. α,β,γ, and δ are used to denote the carbon atoms between which an alkene (C=C) or alkyne (C≡C) bond is present. Scheme 1 below shows the position of the α,β,γ, and δ carbons in a generic ester-containing substrate; this labelling convention is used herein and throughout. Thus, for example, a β,γ unsaturated ester comprises an alkene bond between the β carbon and γ carbon, whilst an α,β-γ,δ unsaturated ester comprises two alkene bonds; that is an alkene bond between the α carbon and β carbon, and an alkene bond between the γ carbon and δ carbon.

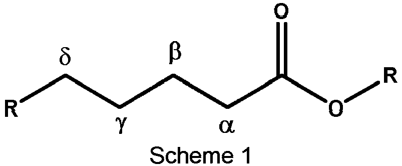

Although a number of processes for ester hydrogenation using transition metal catalysts have been developed, these processes often result in the reduction of alkenyl functional groups, or a change in the regiochemistry of the alkenyl functional groups.

Accordingly, there remains a need for processes capable of selectively reducing unsaturated esters, in particular α,β-γ,δ unsaturated esters and β,γ unsaturated esters, whilst maintaining the regiochemistry of alkenyl functional groups. Document WO 2014/139030 A1 discloses a process for the hydrogenation of methyl-3-nonenoate, a beta,gamma-unsaturated ester. The reaction is done in the presence of the base t-BuOK. Document WO 2019/138216 A1 discloses the hydrogenation of unsaturated esters.

### Summary of the Invention

Accordingly, the present invention provides a process for the hydrogenation of an α,β-γ,δ unsaturated ester or a β,γ unsaturated ester. The process has high chemoselectivity for the hydrogenation of the ester group of α,β-γ,δ unsaturated and β,γ unsaturated esters to the corresponding alcohol, whilst maintaining the regiochemistry and/or conformational geometry of the alkenyl and/or alkynyl functional group(s).

In a first aspect of the invention there is provided a process for the hydrogenation of an ester-containing substrate of Formula (I) to produce an alcohol of Formula (II), 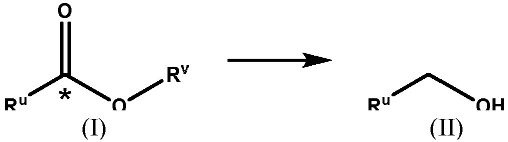 the process comprising treating the ester-containing substrate of Formula (I) with a base and a transition metal catalyst in the presence of molecular hydrogen, wherein:
the ester-containing substrate of Formula (I) comprises an α,β-γ,δ unsaturated ester or a β,γ unsaturated ester;
R^{u} is an organic group having 3-70 carbon atoms, having the proviso that R^{u} is bonded to the carbonyl carbon (*) of the ester moiety to form the α,β-γ,δ unsaturated ester or the β,γ unsaturated ester of Formula (I);
R^{v} is an organic group having 1-70 carbon atoms; and
the conjugate acid of the base has a pKa of from 4 to 15.

It is a surprising advantage of the present invention that the hydrogenation of the α,β-γ,δ unsaturated ester or the β,γ unsaturated ester proceeds without the reduction of the alkenyl functional group(s) of the R^{u} group and that the regiochemistry and/or conformational geometry of the R^{u} group is maintained as between the ester-containing substrate of Formula (I) and the alcohol of Formula (II). In other words, the R^{u} group is the same in the ester-containing substrate of Formula (I) and in the alcohol of Formula (II). The conjugate acid of the base of the invention has a pKa of from 4 to 15, and may be regarded as a weak base. Without wishing to be bound by any sort of theory, it is believed that the use of a base whose conjugate acid has a pKa of from 4 to 15 in the hydrogenation process of the invention prevents conjugation of the carbonyl group and alkenyl functional group(s) and minimises or prevents the formation of enolate intermediates. It is speculated that the formation of such enolate intermediates may be responsible for the reduction of the alkenyl functional group(s) and loss of the alkene regiochemistry. Moreover, it is believed that the formation of enolate intermediates may give rise to the formation of other undesirable by-products such as those resulting from cycloaddition reactions (e.g. Diels-Alder) or condensation reactions. It is further surprising that the use of a base whose conjugate acid has a pKa of from 4 to 15 in the hydrogenation process of the invention actives and maintains the activity of the transition metal catalyst.

Transition metal catalysed reduction of cinnamate esters, which comprise an α,β unsaturated ester, have been reported (for example, de Vries *et. al.,* Adv. Synth. Catal. 2018, 360). However, conditions which may be suitable for hydrogenating cinnamate esters, may not be suitable for ester-containing substrates of Formula (I). In other words, cinnamate esters do not suffer from the same problems as ester-containing substrate of Formula (I), as described hereinabove, as the present inventors do not believe they form enolate intermediates during hydrogenation processes carried out under basic conditions.

### Brief Description of the Drawings

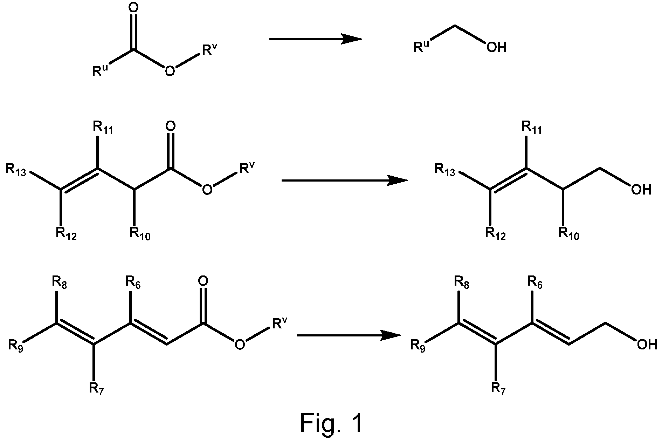

**Figure 1** shows an illustrative reaction scheme for the conversion of ester-containing substrate of Formula (I) to a corresponding alcohol of Formula (II).

### Definitions

The point of attachment of a moiety or substituent is represented by "-". For example, -OH is attached through the oxygen atom.

Unless explicitly stated otherwise, structures depicted herein include all conformational or geometrical isomers of said structure. For example, both E and Z (or *cis* and *trans*) isomers are intended and included where an alkene double bond is shown, and, in the instance where more than one alkene double bond is shown all configurations of conformational or geometrical isomers are included and contemplated (e.g. E,E; E,Z; Z,E; and Z,Z; and cis,cis, cis,trans, trans,cis, and trans,trans).

As used herein, the term "maintenance of regiochemistry" and "retention of regiochemistry" refers to maintenance or retention of the position of any given functional group, for example a β,γ alkene bond.

As used here, the term "geometry" is used to refer to the geometric or conformational configuration of a molecule or functional group, for example the conformation of an E or Z (or cis or trans) alkene bond.

As used herein, the term "alkyl" may include straight-chain or branched saturated hydrocarbon groups. In certain embodiments, the alkyl group may have from 1-20 carbon atoms, in certain embodiments from 1-15 carbon atoms, in certain embodiments, 1-8 carbon atoms. The alkyl group may be unsubstituted. Alternatively, the alkyl group may be substituted. Unless otherwise specified, the alkyl group may be attached at any suitable carbon atom and, if substituted, may be substituted at any suitable atom. Typical alkyl groups include but are not limited to methyl, ethyl, n-propyl, iso-propyl, n-butyl, iso-butyl, sec-butyl, tert-butyl, n-pentyl, n-hexyl and the like.

As used herein, the term "alkenyl" refers to a straight-chain or branched unsaturated hydrocarbon group comprising at least one carbon-carbon double bond.

As used herein, the term "alkynyl" refers to a straight-chain or branched unsaturated hydrocarbon group comprising at least one carbon-carbon triple bond.

As used herein, the term "cycloalkyl" is used to denote a saturated carbocyclic hydrocarbon radical. The cycloalkyl group may have a single ring or multiple condensed rings. In certain embodiments, the cycloalkyl group may have from 3-15 carbon atoms, in certain embodiments, from 3-10 carbon atoms, in certain embodiments, from 3-8 carbon atoms. The cycloalkyl group may be unsubstituted. Alternatively, the cycloalkyl group may be substituted. Unless other specified, the cycloalkyl group may be attached at any suitable carbon atom and, if substituted, may be substituted at any suitable atom. Typical cycloalkyl groups include but are not limited to cyclopropyl, cyclobutyl, cyclopentyl, cyclohexyl, adamantyl and the like.

As used herein, the term "cycloalkenyl" refers to an unsaturated, non-aromatic carbocyclic ring. The cycloalkenyl group therefore has at least one carbon-carbon double bond, but may have more. In certain embodiments, the cycloalkenyl group may have from 3-15 carbon atoms, in certain embodiments, from 3-10 carbon atoms, in certain embodiments, from 3-8 carbon atoms. The cycloalkenyl group may be unsubstituted. Alternatively, the cycloalkenyl group may be substituted. Unless other specified, the cycloalkenyl group may be attached at any suitable carbon atom and, if substituted, may be substituted at any suitable atom. Typical cycloalkenyl groups include but are not limited to cyclopropenyl, cyclobutenyl, cyclopentenyl, cyclohexenyl, and the like.

As used herein, the term "alkoxy" refers to an optionally substituted group of the formula alkyl-O- or cycloalkyl-O-, wherein alkyl and cycloalkyl are as defined above.

As used herein, the term "aryl" refers to an aromatic carbocyclic group. The aryl group may have a single ring or multiple condensed rings. In certain embodiments, the aryl group can have from 6-20 carbon atoms, in certain embodiments from 6-15 carbon atoms, in certain embodiments, 6-12 carbon atoms. The aryl group may be unsubstituted. Alternatively, the aryl group may be substituted. Unless otherwise specified, the aryl group may be attached at any suitable carbon atom and, if substituted, may be substituted at any suitable atom. Examples of aryl groups include, but are not limited to, phenyl, naphthyl, anthracenyl and the like.

As used herein, the term "arylalkyl" refers to an optionally substituted group of the formula aryl-alkyl-, where aryl and alkyl are as defined above.

As used herein, the term "halogen", "halo" or "hal" refers to -F, -CI, -Br and -I.

As used herein, the term "heteroalkyl" refers to a straight-chain or branched saturated hydrocarbon group wherein one or more carbon atoms are independently replaced with one or more heteroatoms (e.g. nitrogen, oxygen, phosphorus and/or sulfur atoms). The heteroalkyl group may be unsubstituted. Alternatively, the heteroalkyl group may be substituted. Unless otherwise specified, the heteroalkyl group may be attached at any suitable atom and, if substituted, may be substituted at any suitable atom. Examples of heteroalkyl groups include but are not limited to ethers, thioethers, primary amines, secondary amines, tertiary amines and the like.

As used herein, the term "heterocycloalkyl" refers to a saturated cyclic hydrocarbon group wherein one or more carbon atoms are independently replaced with one or more heteroatoms (e.g. nitrogen, oxygen, phosphorus and/or sulfur atoms). The heterocycloalkyl group may be unsubstituted. Alternatively, the heterocycloalkyl group may be substituted. Unless otherwise specified, the heterocycloalkyl group may be attached at any suitable atom and, if substituted, may be substituted at any suitable atom. Examples of heterocycloalkyl groups include but are not limited to epoxide, morpholinyl, piperadinyl, piperazinyl, thirranyl, pyrrolidinyl, pyrazolidinyl, imidazolidinyl, thiazolidinyl, thiomorpholinyl and the like.

As used herein, the term "heteroaryl" refers to an aromatic carbocyclic group wherein one or more carbon atoms are independently replaced with one or more heteroatoms (e.g. nitrogen, oxygen, phosphorus and/or sulfur atoms). The heteroaryl group may be unsubstituted. Alternatively, the heteroaryl group may be substituted. Unless otherwise specified, the heteroaryl group may be attached at any suitable atom and, if substituted, may be substituted at any suitable atom. Examples of heteroaryl groups include but are not limited to thienyl, furanyl, pyrrolyl, imidazolyl, pyrazolyl, thiazolyl, isothiazolyl, oxazolyl, isoxazolyl, triazolyl, tetrazolyl, thiadiazolyl, thiophenyl, oxadiazolyl, pyridinyl, pyrimidyl, benzoxazolyl, benzthiazolyl, benzimidazolyl, indolyl, quinolinyl and the like.

As used herein, the term "heterocycle" encompasses both heterocycloalkyl groups and heteroaryl groups.

As used herein, the term "substituted" refers to a group in which one or more hydrogen atoms are each independently replaced with substituents (e.g. 1, 2, 3, 4, 5 or more) which may be the same or different. Examples of substituents include but are not limited to -halo, -C(halo)₃, -R^{c}, =O, =S, -O-R^{c}, -S-R^{c}, -NR^{c}R^{d}, -CN, -NO₂, -C(O)-R^{c}, -COOR^{d} , -C(S)-R^{c}, -C(S)OR^{d}, - S(O)₂OH, -S(O)₂-R^{c}, -S(O)₂NR^{c}R^{d}, -O-S(O)-R^{c} and -CONR^{c}R^{d}, such as -halo, -C(halo)₃ (e.g. -CF₃), -R^{c}, -O-R^{c}, -NR^{c}R^{d}, -CN, or -NO₂. R^{c} and R^{d} are independently selected from the groups consisting of H, alkyl, aryl, arylalkyl, heteroalkyl, heteroaryl, or R^{c} and R^{d} together with the atom to which they are attached form a heterocycloalkyl group. R^{c} and R^{d} may be unsubstituted or further substituted as defined herein.

As used herein, the term "fatty acid" refers to a carboxylic acid with a long aliphatic chain (e.g. >6 carbon atoms), which can be either saturated or unsaturated. The aliphatic chain of the fatty acid may be branched or unbranched. In certain embodiments, the aliphatic chain of the fatty acid comprises 12 to 24 carbon atoms. In certain embodiments, the aliphatic chain of the fatty acid comprises 0 to 5 carbon-carbon double bonds.

As used herein, the term "fatty alcohol" refers to an alcohol with a long aliphatic chain (e.g. >6 carbon atoms), which can be either saturated or unsaturated. The aliphatic chain of the fatty alcohol may be branched or unbranched. In certain embodiments, the aliphatic chain of the fatty alcohol comprises 12 to 24 carbon atoms. In certain embodiments, the aliphatic chain of the fatty alcohol comprises 0 to 5 carbon-carbon double bonds.

As used herein, the terms "wax ester" or "waxy ester" refer to an ester of a fatty acid and a fatty alcohol, wherein fatty acid and fatty alcohol are as defined above.

As used herein, the term "bidentate ligand" refers to a ligand that donates two pairs of electrons to a metal atom.

As used herein, the term "tridentate ligand" refers to a ligand that donates three pairs of electrons to a metal atom.

As used herein, the term "tetradentate ligand" refers to a ligand that donates four pairs of electrons to a metal atom.

As used herein, the term "Ru-SNS" refers to dichlorotriphenylphosphine[bis(2-(ethylthio)ethyl)amine]ruthenium(II).

As used herein, the term "Ru-PNN" refers to dichlorotriphenylphosphine[2-(diphenylphosphino)-N-(2-pyridinylmethyl)ethanamine]ruthenium(ll).

As used herein, the term "Ru-SNN" refers to dichlorotriphenylphosphine[2-(ethylthio)-N-(2-pyridinylmethyl)ethanamine]ruthenium(II).

As used herein, the term "S/C" is an abbreviation for "substrate/catalyst" and is used to describe the catalyst loading employed in a reaction, i.e. it describes the molar ratio of ester-containing substrate and catalyst present in the reaction mixture. In the instance the ester-containing substrate contains more than one ester moiety, the S/C value is adjusted accordingly. For example, a molar ratio of triglyceride to catalyst of 10,000:1 equates to an S/C of 30,000:1 (as a triglyceride substrate contains three ester moieties).

As used herein, the term "turnover number" (TON) refers to the number of moles of substrate that a mole of catalyst can convert before becoming deactivated.

As used herein, unless otherwise specified, "mol%" describes the amount of moles of the specified material (e.g. a base) relative to the amount of moles of the ester-containing substrate, as a percentage. The "mol%" amount given for the specified material (e.g. a base) is the amount of that material employed in the reaction chamber (i.e. the location where the hydrogenation reaction takes place).

As used herein, the term "hydrogenation" refers to hydrogenation using molecular hydrogen.

### Detailed Description

Preferred and/or optional features of the invention will now be set out. The invention is set out in the claims.

The present invention provides a process for the hydrogenation of an ester-containing substrate of Formula (I) to produce an alcohol of Formula (II), 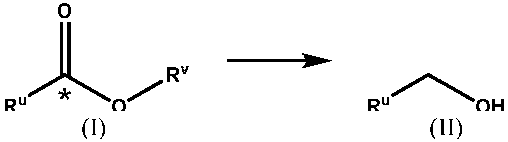 the process comprising treating the ester-containing substrate of Formula (I) with a base and a transition metal catalyst in the presence of molecular hydrogen, wherein:
the ester-containing substrate of Formula (I) comprises an α,β-γ,δ unsaturated ester or a β,γ unsaturated ester;
R^{u} is an organic group having 3-70 carbon atoms, having the proviso that R^{u} is bonded to the carbonyl carbon (*) of the ester moiety to form the α,β-γ,δ unsaturated ester or the β,γ unsaturated ester of Formula (I);
R^{v} is an organic group having 1-70 carbon atoms; and
the conjugate acid of the base has a pKa of from 4 to 15.

### Ester-containing Substrate

The process of the invention comprises the hydrogenation of an ester-containing substrate of Formula (I), wherein the ester-containing substrate of Formula (I) comprises an α,β-γ,δ unsaturated ester or a β,γ unsaturated ester. The ester-containing substrate of Formula (I) contains at least one ester moiety.

In preferred processes of the invention the ester-containing substrate of Formula (I) comprises an α,β-γ,δ unsaturated ester. In preferred processes of the invention the ester-containing substrate of Formula (I) comprises a β,γ unsaturated ester.

In preferred processes of the invention the ester-containing substrate of Formula (I) comprises an α,β-γ,δ unsaturated ester or a β,γ unsaturated ester and the bond between the α and β carbon atoms and the γ and δ carbon atoms in the α,β-γ,δ unsaturated ester, or the β and γ carbon atoms in the β,γ unsaturated ester are each alkenyl bonds. In the instance where the ester-containing substrate of Formula (I) is an α,β-γ,δ unsaturated ester, it may be preferred that the α,β-γ,δ unsaturated ester has a cis,cis geometry, a cis,trans geometry, a trans,cis geometry, or a trans,trans geometry. In the instance where the ester-containing substrate of Formula (I) is a β,γ unsaturated ester, it may be preferred that the β,γ unsaturated ester has a cis geometry or a trans geometry.

The ester-containing substrate is of Formula (I): 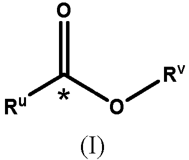 wherein:
R^{u} is an organic group having 3-70 carbon atoms, having the proviso that R^{u} is bonded to the carbonyl carbon (*) of the ester moiety to form the α,β-δ,γ unsaturated ester or the β,δ unsaturated ester of Formula (I); and
R^{v} is an organic group having 1-70 carbon atoms.

As will be understood, R^{u} is bonded to the carbonyl carbon (*) of the ester moiety of the ester-containing substrate of Formula (I) to form the α,β-γ,δ unsaturated ester or the β,γ unsaturated ester.

In preferred processes of the invention, R^{u} does not comprise a terminal CH₂ group having a sp² hybridised carbon atom. Thus, in preferred processes of the invention, there is the proviso that R^{u} is bonded to the carbonyl carbon (*) of the ester moiety to form the α,β-δ,γ unsaturated ester or the β,δ unsaturated ester of Formula (I), and R^{u} does not comprise a terminal CH₂ group having a sp² hybridised carbon atom.

Preferably, R^{u} is an organic group having Formula (XII): 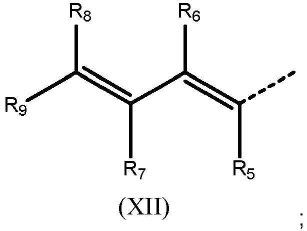 wherein the dashed bond indicates the bond to the carbonyl carbon (*) of the ester moiety of the ester-containing substrate of Formula (I).

In the instance where R^{u} is an organic group having Formula (XII) the ester-containing substrate of Formula (I) comprises an α,β-γ,δ unsaturated ester.

R₅-R₈ are each independently a hydrogen atom or an organic group having 1-70 carbon atoms.

In preferred processes of the invention, R₅-R₈ are each independently selected from a hydrogen atom, substituted or unsubstituted C₁₋₇₀-alkyl, substituted or unsubstituted C₂₋₇₀-alkenyl, substituted or unsubstituted C₂₋₇₀-alkynyl, substituted or unsubstituted C₁₋₇₀-heteroalkyl, substituted or unsubstituted C₃₋₇₀-cycloalkyl, substituted or unsubstituted C₃₋₇₀-cycloalkenyl, substituted or unsubstituted C₂₋₇₀-heterocycloalkyl, substituted or unsubstituted C₆₋₇₀-aryl, and substituted or unsubstituted C₄₋₇₀-heteroaryl, preferably substituted or unsubstituted C₁₋₅₀-alkyl, substituted or unsubstituted C₂₋₅₀-alkenyl, substituted or unsubstituted C₂₋₅₀-alkynyl, substituted or unsubstituted C₁₋₅₀-heteroalkyl, substituted or unsubstituted C₃₋₅₀-cycloalkyl, substituted or unsubstituted C₃₋₅₀-cycloalkenyl, substituted or unsubstituted C₂₋₅₀-heterocycloalkyl, substituted or unsubstituted C₆₋₅₀-aryl, and substituted or unsubstituted C₄₋₅₀-heteroaryl, more preferably substituted or unsubstituted C₁₋₃₀-alkyl, substituted or unsubstituted C₂₋₃₀-alkenyl, substituted or unsubstituted C₂₋₃₀-alkynyl, substituted or unsubstituted C₁₋₃₀-heteroalkyl, substituted or unsubstituted C₃₋₃₀-cycloalkyl, substituted or unsubstituted C₃₋₃₀-cycloalkenyl, substituted or unsubstituted C₂₋₃₀-heterocycloalkyl, substituted or unsubstituted C₆₋₃₀-aryl, and substituted or unsubstituted C₄₋₃₀-heteroaryl, even more preferably substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₂₋₂₀-alkynyl (e.g. C₈₋₂₀-alkynyl), substituted or unsubstituted C₁₋₂₀-heteroalkyl (e.g. C₈₋₂₀-heteroalkyl), substituted or unsubstituted C₃₋₂₀-cycloalkyl (e.g. C₈₋₂₀-cycloalkyl), substituted or unsubstituted C₃₋₂₀-cycloalkenyl (e.g. C₈₋₂₀-cycloalkenyl), substituted or unsubstituted C₂₋₂₀-heterocycloalkyl (e.g. C₈₋₂₀-heterocycloalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). Preferably, R5-R8 are each independently selected from a hydrogen atom, substituted or unsubstituted C₁₋₇₀-alkyl, substituted or unsubstituted C₂₋₇₀-alkenyl, substituted or unsubstituted C₁₋₇₀-heteroalkyl, substituted or unsubstituted C₆₋₇₀-aryl, and substituted or unsubstituted C₄₋₇₀-heteroaryl, more preferably substituted or unsubstituted C₁₋₅₀-alkyl, substituted or unsubstituted C₂₋₅₀-alkenyl, substituted or unsubstituted C₁₋₅₀-heteroalkyl, substituted or unsubstituted C₆₋₅₀-aryl, and substituted or unsubstituted C₄₋₅₀-heteroaryl, even more preferably substituted or unsubstituted C₁₋₃₀-alkyl, substituted or unsubstituted C₂₋₃₀-alkenyl, substituted or unsubstituted C₁₋₃₀-heteroalkyl, substituted or unsubstituted C₆₋₃₀-aryl, and substituted or unsubstituted C₄₋₃₀-heteroaryl, even more preferably substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₁₋₂₀-heteroalkyl (e.g. C₈₋₂₀-heteroalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). More preferably, R₅-R₈ are each independently selected from a hydrogen atom, substituted or unsubstituted C₁₋₇₀-alkyl, substituted or unsubstituted C₂₋₇₀-alkenyl, and substituted or unsubstituted C₆₋₇₀-aryl, more preferably a hydrogen atom, substituted or unsubstituted C₁₋₅₀-alkyl, substituted or unsubstituted C₂₋₅₀-alkenyl, and substituted or unsubstituted C₆₋₅₀-aryl, even more preferably a hydrogen atom, substituted or unsubstituted C₁₋₃₀-alkyl, substituted or unsubstituted C₂₋₃₀-alkenyl, and substituted or unsubstituted C₆₋₃₀-aryl, even more preferably a hydrogen atom, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), and substituted or unsubstituted C₆₋₂₀-aryl (e.g. C₈₋₂₀-aryl). More preferably, R₅-R₈ are each independently selected from a hydrogen atom, substituted or unsubstituted C₁₋₇₀-alkyl and substituted or unsubstituted C₆₋₇₀-aryl, more preferably a hydrogen atom, substituted or unsubstituted C₁₋₅₀-alkyl and substituted or unsubstituted C₆₋₅₀-aryl, even more preferably a hydrogen atom, substituted or unsubstituted C₁₋₃₀-alkyl and substituted or unsubstituted C₆₋₃₀-aryl, even more preferably a hydrogen atom, substituted or unsubstituted C₁₋₂₀-alkyl (e.g. C₈₋₂₀-alkyl) and substituted or unsubstituted C₆₋₂₀-aryl (e.g. C₈₋₂₀-aryl). Even more preferably, R₅-R₈ are each independently selected from a hydrogen atom, methyl, ethyl, n-propyl, iso-propyl, n-butyl, sec-butyl, tert-butyl, pentyl, hexyl, heptyl, octyl, phenyl, tolyl, xylyl, and methoxyphenyl. Most preferably, R₅-R₈ are hydrogen atoms. Alternatively, two of R₅, R₆, and R₇ or two of R₆, R₇, and R₈ together with the atoms to which they are attached form a substituted or unsubstituted cycloalkyl ring, a substituted or unsubstituted cycloalkenyl ring, or a substituted or unsubstituted heterocycloalkyl ring, for example an alkyl cyclohex-2-ene-1-carboxylate or an alkyl cyclopentene-1-carboxylate.

R₉ is H or an organic group having 1-70 carbon atoms. Preferably, R₉ is H, a substituted or unsubstituted C₁₋₇₀-alkyl, substituted or unsubstituted C₂₋₇₀-alkenyl, substituted or unsubstituted C₂₋₇₀-alkynyl, substituted or unsubstituted C₁₋₇₀-heteroalkyl, substituted or unsubstituted C₃₋₇₀-cycloalkyl, substituted or unsubstituted C₃₋₇₀-cycloalkenyl, substituted or unsubstituted C₂₋₇₀-heterocycloalkyl, substituted or unsubstituted C₆₋₇₀-aryl, and substituted or unsubstituted C₄₋₇₀-heteroaryl, preferably H, substituted or unsubstituted C₁₋₅₀-alkyl, substituted or unsubstituted C₂₋₅₀-alkenyl, substituted or unsubstituted C₂₋₅₀-alkynyl, substituted or unsubstituted C₁₋₅₀-heteroalkyl, substituted or unsubstituted C₃₋₅₀-cycloalkyl, substituted or unsubstituted C₃₋₅₀-cycloalkenyl, substituted or unsubstituted C₂₋₅₀-heterocycloalkyl, substituted or unsubstituted C₆₋₅₀-aryl, and substituted or unsubstituted C₄₋₅₀-heteroaryl, more preferably H, substituted or unsubstituted C₁₋₃₀-alkyl, substituted or unsubstituted C₂₋₃₀-alkenyl, substituted or unsubstituted C₂₋₃₀-alkynyl, substituted or unsubstituted C₁₋₃₀-heteroalkyl, substituted or unsubstituted C₃₋₃₀-cycloalkyl, substituted or unsubstituted C₃₋₃₀-cycloalkenyl, substituted or unsubstituted C₂₋₃₀-heterocycloalkyl, substituted or unsubstituted C₆₋₃₀-aryl, and substituted or unsubstituted C₄₋₃₀-heteroaryl, even more preferably H, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₂₋₂₀-alkynyl (e.g. C₈₋₂₀-alkynyl), substituted or unsubstituted C₁₋₂₀-heteroalkyl (e.g. C₈₋₂₀-heteroalkyl), substituted or unsubstituted C₃₋₂₀-cycloalkyl (e.g. C₈₋₂₀-cycloalkyl), substituted or unsubstituted C₃₋₂₀-cycloalkenyl (e.g. C₈₋₂₀-cycloalkenyl), substituted or unsubstituted C₂₋₂₀-heterocycloalkyl (e.g. C₈₋₂₀-heterocycloalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). Preferably, R₉ is selected from H, substituted or unsubstituted C₁₋₇₀-alkyl, substituted or unsubstituted C₂₋₇₀-alkenyl, substituted or unsubstituted C₁₋₇₀-heteroalkyl, substituted or unsubstituted C₆₋₇₀-aryl, and substituted or unsubstituted C₄₋₇₀-heteroaryl, more preferably H, substituted or unsubstituted C₁₋₅₀-alkyl, substituted or unsubstituted C₂₋₅₀-alkenyl, substituted or unsubstituted C₁₋₅₀-heteroalkyl, substituted or unsubstituted C₆₋₅₀-aryl, and substituted or unsubstituted C₄₋₅₀-heteroaryl, even more preferably H, substituted or unsubstituted C₁₋₃₀-alkyl, substituted or unsubstituted C₂₋₃₀-alkenyl, substituted or unsubstituted C₁₋₃₀-heteroalkyl, substituted or unsubstituted C₆₋₃₀-aryl, and substituted or unsubstituted C₄₋₃₀-heteroaryl, even more preferably H, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₁₋₂₀-heteroalkyl (e.g. C₈₋₂₀-heteroalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). More preferably, R₉ is selected from H, substituted or unsubstituted C₁₋₇₀-alkyl, substituted or unsubstituted C₂₋₇₀-alkenyl, and substituted or unsubstituted C₆₋₇₀-aryl, more preferably H, substituted or unsubstituted C₁₋₅₀-alkyl, substituted or unsubstituted C₂₋₅₀-alkenyl, and substituted or unsubstituted C₆₋₅₀-aryl, even more preferably H, substituted or unsubstituted C₁₋₃₀-alkyl, substituted or unsubstituted C₂₋₃₀-alkenyl, and substituted or unsubstituted C₆₋₃₀-aryl, even more preferably H, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), and substituted or unsubstituted C₆₋₂₀-aryl (e.g. C₈₋₂₀-aryl). More preferably, R₉ is selected from H, a substituted or unsubstituted C₁₋₇₀-alkyl and substituted or unsubstituted C₆₋₇₀-aryl, more preferably H, substituted or unsubstituted C₁₋₅₀-alkyl and substituted or unsubstituted C₆₋₅₀-aryl, even more preferably H, substituted or unsubstituted C₁₋₃₀-alkyl and substituted or unsubstituted C₆₋₃₀-aryl, even more preferably H, substituted or unsubstituted C₁₋₂₀-alkyl (e.g. C₈₋₂₀-alkyl) and substituted or unsubstituted C₆₋₂₀-aryl (e.g. C₈₋₂₀-aryl). Even more preferably, R₉ is selected from H, methyl, ethyl, n-propyl, iso-propyl, n-butyl, sec-butyl, tert-butyl, pentyl, hexyl, heptyl, octyl, phenyl, tolyl, xylyl, and methoxyphenyl. Most preferably, R₉ is selected from methyl, ethyl, and n-propyl.

Alternatively, R^{u} may be an organic group having Formula (XIII): 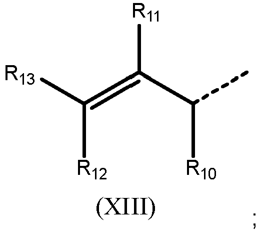 wherein the dashed bond indicates the bond to the carbonyl carbon (*) of the ester moiety of the ester-containing substrate of Formula (I).

In the instance where R^{u} is an organic group of Formula (XIII) the ester-containing substrate of Formula (I) comprises a β,γ unsaturated ester.

R₁₀-R₁₂ are each independently a hydrogen atom or an organic group having 1-70 carbon atoms.

In preferred processes of the invention, R₁₀-R₁₂ are each independently selected from a hydrogen atom, substituted or unsubstituted C₁₋₇₀-alkyl, substituted or unsubstituted C₂₋₇₀-alkenyl, substituted or unsubstituted C₂₋₇₀-alkynyl, substituted or unsubstituted C₁₋₇₀-heteroalkyl, substituted or unsubstituted C₃₋₇₀-cycloalkyl, substituted or unsubstituted C₃₋₇₀-cycloalkenyl, substituted or unsubstituted C₂₋₇₀-heterocycloalkyl, substituted or unsubstituted C₆₋₇₀-aryl, and substituted or unsubstituted C₄₋₇₀-heteroaryl, preferably substituted or unsubstituted C₁₋₅₀-alkyl, substituted or unsubstituted C₂₋₅₀-alkenyl, substituted or unsubstituted C₂₋₅₀-alkynyl, substituted or unsubstituted C₁₋₅₀-heteroalkyl, substituted or unsubstituted C₃₋₅₀-cycloalkyl, substituted or unsubstituted C₃₋₅₀-cycloalkenyl, substituted or unsubstituted C₂₋₅₀-heterocycloalkyl, substituted or unsubstituted C₆₋₅₀-aryl, and substituted or unsubstituted C₄₋₅₀-heteroaryl, more preferably substituted or unsubstituted C₁₋₃₀-alkyl, substituted or unsubstituted C₂₋₃₀-alkenyl, substituted or unsubstituted C₂₋₃₀-alkynyl, substituted or unsubstituted C₁₋₃₀-heteroalkyl, substituted or unsubstituted C₃₋₃₀-cycloalkyl, substituted or unsubstituted C₃₋₃₀-cycloalkenyl, substituted or unsubstituted C₂₋₃₀-heterocycloalkyl, substituted or unsubstituted C₆₋₃₀-aryl, and substituted or unsubstituted C₄₋₃₀-heteroaryl, even more preferably substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₂₋₂₀-alkynyl (e.g. C₈₋₂₀-alkynyl), substituted or unsubstituted C₁₋₂₀-heteroalkyl (e.g. C₈₋₂₀-heteroalkyl), substituted or unsubstituted C₃₋₂₀-cycloalkyl (e.g. C₈₋₂₀-cycloalkyl), substituted or unsubstituted C₃₋₂₀-cycloalkenyl (e.g. C₈₋₂₀-cycloalkenyl), substituted or unsubstituted C₂₋₂₀-heterocycloalkyl (e.g. C₈₋₂₀-heterocycloalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). Preferably, R₁₀-R₁₂ are each independently selected from a hydrogen atom, substituted or unsubstituted C₁₋₇₀-alkyl, substituted or unsubstituted C₂₋₇₀-alkenyl, substituted or unsubstituted C₁₋₇₀-heteroalkyl, substituted or unsubstituted C₆₋₇₀-aryl, and substituted or unsubstituted C₄₋₇₀-heteroaryl, more preferably substituted or unsubstituted C₁₋₅₀-alkyl, substituted or unsubstituted C₂₋₅₀-alkenyl, substituted or unsubstituted C₁₋₅₀-heteroalkyl, substituted or unsubstituted C₆₋₅₀-aryl, and substituted or unsubstituted C₄₋₅₀-heteroaryl, even more preferably substituted or unsubstituted C₁₋₃₀-alkyl, substituted or unsubstituted C₂₋₃₀-alkenyl, substituted or unsubstituted C₁₋₃₀-heteroalkyl, substituted or unsubstituted C₆₋₃₀-aryl, and substituted or unsubstituted C₄₋₃₀-heteroaryl, even more preferably substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₁₋₂₀-heteroalkyl (e.g. C₈₋₂₀-heteroalkyl), substituted or unsubstituted C₆₋₂₀-aryl (e.g. C₈₋₂₀-aryl), and substituted or unsubstituted C₄₋₂₀-heteroaryl (e.g. C₈₋₂₀-heteroaryl). More preferably, R₁₀-R₁₂ are each independently selected from a hydrogen atom, substituted or unsubstituted C₁₋₇₀-alkyl, substituted or unsubstituted C₂₋₇₀-alkenyl, and substituted or unsubstituted C₆₋₇₀-aryl, more preferably a hydrogen atom, substituted or unsubstituted C₁₋₅₀-alkyl, substituted or unsubstituted C₂₋₅₀-alkenyl, and substituted or unsubstituted C₆₋₅₀-aryl, even more preferably a hydrogen atom, substituted or unsubstituted C₁₋₃₀-alkyl, substituted or unsubstituted C₂₋₃₀-alkenyl, and substituted or unsubstituted C₆₋₃₀-aryl, even more preferably a hydrogen atom, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), and substituted or unsubstituted C₆₋₂₀-aryl (e.g. C₈₋₂₀-aryl). More preferably, R₁₀-R₁₂ are each independently selected from a hydrogen atom, substituted or unsubstituted C₁₋₇₀-alkyl and substituted or unsubstituted C₆₋₇₀-aryl, more preferably a hydrogen atom, substituted or unsubstituted C₁₋₅₀-alkyl and substituted or unsubstituted C₆₋₅₀-aryl, even more preferably a hydrogen atom, substituted or unsubstituted C₁₋₃₀-alkyl and substituted or unsubstituted C₆₋₃₀-aryl, even more preferably a hydrogen atom, substituted or unsubstituted C₁₋₂₀-alkyl (e.g. C₈₋₂₀-alkyl) and substituted or unsubstituted C₆₋₂₀-aryl (e.g. C₈₋₂₀-aryl). Even more preferably, R₁₀-R₁₂ are each independently selected from a hydrogen atom, methyl, ethyl, n-propyl, iso-propyl, n-butyl, sec-butyl, tert-butyl, pentyl, hexyl, heptyl, octyl, phenyl, tolyl, xylyl, and methoxyphenyl. Most preferably, R₁₀-R₁₂ are hydrogen atoms. Alternatively, two of R₁₀, R₁₁, and R₁₂ together with the atoms to which they are attached form a substituted or unsubstituted cycloalkyl ring, a substituted or unsubstituted cycloalkenyl ring, or a substituted or unsubstituted heterocycloalkyl ring.

R₁₃ is H or an organic group having 1-70 carbon atoms. Preferably, R₁₃ is H, a substituted or unsubstituted C₁₋₇₀-alkyl, substituted or unsubstituted C₂₋₇₀-alkenyl, substituted or unsubstituted C₂₋₇₀-alkynyl, substituted or unsubstituted C₁₋₇₀-heteroalkyl, substituted or unsubstituted C₃₋₇₀-cycloalkyl, substituted or unsubstituted C₃₋₇₀-cycloalkenyl, and substituted or unsubstituted C₂₋₇₀-heterocycloalkyl, , preferably H, substituted or unsubstituted C₁₋₅₀-alkyl, substituted or unsubstituted C₂₋₅₀-alkenyl, substituted or unsubstituted C₂₋₅₀-alkynyl, substituted or unsubstituted C₁₋₅₀-heteroalkyl, substituted or unsubstituted C₃₋₅₀-cycloalkyl, substituted or unsubstituted C₃₋₅₀-cycloalkenyl, and substituted or unsubstituted C₂₋₅₀-heterocycloalkyl, , more preferably H, substituted or unsubstituted C₁₋₃₀-alkyl, substituted or unsubstituted C₂₋₃₀-alkenyl, substituted or unsubstituted C₂₋₃₀-alkynyl, substituted or unsubstituted C₁₋₃₀-heteroalkyl, substituted or unsubstituted C₃₋₃₀-cycloalkyl, substituted or unsubstituted C₃₋₃₀-cycloalkenyl, and substituted or unsubstituted C₂₋₃₀-heterocycloalkyl, , even more preferably H, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), substituted or unsubstituted C₂₋₂₀-alkynyl (e.g. C₈₋₂₀-alkynyl), substituted or unsubstituted C₁₋₂₀-heteroalkyl (e.g. C₈₋₂₀-heteroalkyl), substituted or unsubstituted C₃₋₂₀-cycloalkyl (e.g. C₈₋₂₀-cycloalkyl), substituted or unsubstituted C₃₋₂₀-CyCloalkenyl (e.g. C₈₋₂₀-cycloalkenyl), and substituted or unsubstituted C₂₋₂₀-heterocycloalkyl (e.g. C₈₋₂₀-heterocycloalkyl). Preferably, R₁₃ is selected from H, substituted or unsubstituted C₁₋₇₀-alkyl, and substituted or unsubstituted C₂₋₇₀-alkenyl, substituted or unsubstituted C₁₋₇₀-heteroalkyl, , more preferably H, substituted or unsubstituted C₁₋₅₀-alkyl, substituted or unsubstituted C₂₋₅₀-alkenyl, and substituted or unsubstituted C₁₋₅₀-heteroalkyl, even more preferably substituted or unsubstituted C₁₋₃₀-alkyl, substituted or unsubstituted C₂₋₃₀-alkenyl, and substituted or unsubstituted C₁₋₃₀-heteroalkyl, even more preferably H, substituted or unsubstituted C₁₋₂₀-alkyl (e.g. C₈₋₂₀-alkyl), substituted or unsubstituted C₂₋₂₀-alkenyl (e.g. C₈₋₂₀-alkenyl), and substituted or unsubstituted C₁₋₂₀-heteroalkyl (e.g. C₈₋₂₀-heteroalkyl). More preferably, R₁₃ is selected from H, substituted or unsubstituted C₁₋₇₀-alkyl, substituted or unsubstituted C₂₋₇₀-alkenyl, and substituted or unsubstituted C₆₋₇₀-aryl, more preferably H, substituted or unsubstituted C₁₋₅₀-alkyl, and substituted or unsubstituted C₂₋₅₀-alkenyl, even more preferably H, substituted or unsubstituted C₁₋₃₀-alkyl, and substituted or unsubstituted C₂₋₃₀-alkenyl, even more preferably substituted or unsubstituted C₁₋₂₀-alkyl (e.g. C₈₋₂₀-alkyl), and substituted or unsubstituted C₂₋₂₀-alkenyl (e.g. C₈₋₂₀-alkenyl). More preferably, R₁₃ is selected from H, a substituted or unsubstituted C₁₋₇₀-alkyl, more preferably substituted or unsubstituted C₁₋₅₀-alkyl, even more preferably H, substituted or unsubstituted C₁₋₃₀-alkyl, even more preferably substituted or unsubstituted C₁₋₂₀-alkyl (e.g. C₈₋₂₀-alkyl). Even more preferably, R₁₃ is selected from H, methyl, ethyl, n-propyl, iso-propyl, n-butyl, sec-butyl, tert-butyl, pentyl, hexyl, heptyl, octyl, phenyl, tolyl, xylyl, and methoxyphenyl. Most preferably, R₁₃ is selected from methyl, ethyl, and n-propyl.

It may be preferred that R₁₃ is not a terminal CH₂ group having a sp² hybridised carbon atom. It may be preferred that R₉ is not a terminal CH₂ having a sp² hybridised carbon atom. It may be preferred that R₉ and R₁₃ are not terminal CH₂ groups having sp² hybridised carbon atoms.

In the ester-containing substrate of Formula (I), R^{v} is an organic group having 1-70 carbon atoms. In preferred processes of the invention, R^{v} is selected from substituted or unsubstituted C₁₋₇₀-alkyl, preferably substituted or unsubstituted C₁₋₅₀-alkyl, more preferably substituted or unsubstituted C₁₋₃₀-alkyl, even more preferably substituted or unsubstituted C₁₋₂₀-alkyl (e.g. C₂₋₂₀-alkyl or C₃₋₂₀-alkyl). Preferably, R^{v} is selected from substituted or unsubstituted C₂₋₇₀-alkyl, more preferably substituted or unsubstituted C₂₋₅₀-alkyl, even more preferably substituted or unsubstituted C₂₋₃₀-alkyl, even more preferably substituted or unsubstituted C₁₋₂₀-alkyl, C₂₋₂₀-alkyl, or C₃₋₂₀-alkyl. More preferably, R^{v} is selected from methyl, ethyl, n-propyl, iso-propyl, n-butyl, sec-butyl, iso-butyl, tert-butyl, pentyl, hexyl, heptyl, and octyl. More preferably still, R^{v} is selected from methyl, ethyl, n-propyl, or iso-propyl. Even more preferably, R^{v} is ethyl, n-butyl, or tert-butyl. Most preferably, R^{v} is ethyl.

In preferred processes of the invention, the ester-containing substrate of Formula (I) is an alkyl sorbate. Preferably, the alkyl sorbate is methyl sorbate, ethyl sorbate, n-propyl sorbate, iso-propyl sorbate, n-butyl sorbate, sec-butyl sorbate, or tert-butyl sorbate. More preferably, the alkyl sorbate is methyl sorbate or ethyl sorbate.

In preferred processes of the invention, the ester-containing substate of Formula (I) is an alkyl trans-3-hexanoate. Preferably, the alkyl trans-3-hexanoate is methyl trans-3-hexanoate, ethyl trans-3-hexanoate, n-propyl trans-3-hexanoate, iso-propyl trans-3-hexanoate, n-butyl trans-3-hexanoate, sec-butyl trans-3-hexanoate, iso-butyl trans-3-hexanoate, or tert-butyl trans-3-hexanoate. More preferably the alkyl trans-3-hexanoate is methyl trans-3-hexanoate or ethyl trans-3-hexanoate.

In preferred processes of the invention, the ester-containing substrate of Formula (I) is an alkyl cis-3-hexanoate. Preferably, the alkyl cis-3-hexanoate is methyl cis -3-hexanoate, ethyl cis-3-hexanoate, n-propyl cis-3-hexanoate, iso-propyl cis-3-hexanoate, n-butyl cis-3-hexanoate, sec-butyl cis-3-hexanoate, iso-butyl cis-3-hexanoate, or tert-butyl cis-3-hexanoate. More preferably the alkyl cis-3-hexanoate is methyl cis-3-hexanoate or ethyl cis-3-hexanoate.

In preferred process of the invention, the ester-containing substrate of Formula (I) is an alkyl sorbate, an alkyl trans-3-hexanoate, or an alkyl cis-3-hexanoate as defined hereinabove.

### Alcohol Products

When the ester-containing substrate of Formula (I) is a monoester, the products of the process of the invention is an alcohol of Formula (II). In preferred processes of the invention, the conformational geometry of the alkenyl groups in the group R^{u} are the same in the ester-containing substrate of Formula(I) and the product alcohol of Formula (II).

In preferred processes of the invention the alcohol of Formula (II) is trans,trans hexa-2,4-diene-1-ol:

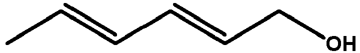

Accordingly, in preferred embodiments of the invention, the process is a process for the hydrogenation of an ester-containing substrate of Formula (I) to produce an alcohol of Formula (II) which is trans,trans hexa-2,4-diene-1-ol.

In a most preferred processes of the invention the ester-containing substrate of Formula (I) is an alkyl sorbate as defined hereinabove, and the alcohol of Formula (II) is trans,trans hexa-2,4-diene-1-ol.

In preferred processes of the invention the alcohol of Formula (II) is trans-hexa-3-ene-1-ol:

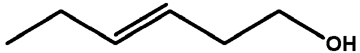

Accordingly, in preferred embodiments of the invention, the process is a process for the hydrogenation of an ester-containing substrate of Formula (I) to produce an alcohol of Formula (II) which is which is trans-hexa-3-ene-1-ol.

In a most preferred processes of the invention the ester-containing substrate of Formula (I) is an alkyl trans-3-hexanoate as defined hereinabove, and the alcohol of Formula (II) is trans-hexa-3-ene-1-ol.

In preferred processes of the invention the alcohol of Formula (II) is cis-hexa-3-ene-1-ol:

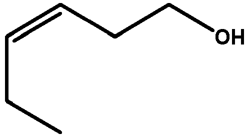

Accordingly, in preferred embodiments of the invention, the process is a process for the hydrogenation of an ester-containing substrate of Formula (I) to produce an alcohol of Formula (II) which is which is cis-hexa-3-ene-1-ol.

In a most preferred processes of the invention the ester-containing substrate of Formula (I) is an alkyl cis-3-hexanoate as defined hereinabove, and the alcohol of Formula (II) is cis-hexa-3-ene-1-ol.

### Base

The base of the present invention is one whose conjugate acid has a pKa of from 4 to 15. Without wishing to be bound by any sort of theory, it is believed that the use of a base whose conjugate acid has a pKa of from 4 to 15 minimises or prevents the formation of enolate intermediates in the hydrogenation process of the invention. It is speculated that the formation of such enolate intermediates may be responsible for the reduction of the alkenyl functional group(s) and loss of the alkene regiochemistry. Furthermore, it is believed that the formation of enolate intermediates may give rise to the formation of other undesirable by-products such as those resulting from cycloaddition reactions (e.g. Diels-Alder) or condensation reactions. Moreover, it is believed that the base plays a role in the activation of the transition metal catalyst. It is therefore surprising that the base, whose conjugate acid has a pKa of from 4 to 15, functions to both activate the transition metal catalyst of the invention whilst minimising or preventing formation of enolate intermediates.

In preferred processes of the invention the conjugate acid of the base has a pKa of greater than 5, greater than 7, or greater than 9. In preferred processes of the invention the conjugate acid of the base has a pKa of less than 14, less than 13, or less than 12. In preferred processes of the invention the conjugate acid of the base has a pKa of from 5 to 14, from 7 to 13, or from 9 to 12, for example 9 to 11, for example about 10.

In preferred processes of the invention, the base is a metal phosphate or a metal carbonate wherein the conjugate acid of the base has a pKa of from 4 to 15. The metal carbonate or the metal phosphate is preferably an alkali metal phosphate, an alkali earth metal phosphate, an alkali metal carbonate, or an alkali earth metal carbonate wherein the conjugate acid of the base has a pKa of from 4 to 15.

In more preferred processes of the invention, the base is a metal phosphate wherein the conjugate acid of the base has a pKa of from 4 to 15. The metal phosphate is preferably an alkali metal phosphate or an alkali earth metal phosphate wherein the conjugate acid of the base has a pKa of from 4 to 15. The metal phosphate is more preferably an alkali metal phosphate. The alkali metal phosphate is preferably lithium phosphate (Li₃PO₄), sodium phosphate (Na₃PO₄), potassium phosphate (K₃PO₄), or caesium phosphate (Cs₃PO₄). Most preferably, the base is an alkali metal phosphate which is potassium phosphate (K₃PO₄).

In preferred processes of the invention, the base is present in solid form.

In preferred processes of the invention, the base is present in at least 30 mol% based upon the total amount of ester-containing substrate, preferably in at least 35 mol% based upon the total amount of ester-containing substrate, more preferably in at least 40 mol% based upon the total amount of ester-containing substrate, and even more preferably in at least 45 mol% based upon the total amount of ester-containing substrate.

In preferred processes of the invention, the base is present in less than or equal to 200 mol% based upon the total amount of ester-containing substrate, more preferably in less than or equal to 125 mol% based upon the total amount of ester-containing substrate.

In preferred processes of the invention, the base is present in a range of 30 to 70 mol% based upon the total amount of ester-containing substrate, more preferably in a range of 30 to 60 mol% based upon the total amount of ester-containing substrate, even more preferably in a range of 30 to 50 mol% based upon the total amount of ester-containing substrate.

In preferred processes of the invention the base is present in the process as a solid. In preferred processes of the invention the base is substantially insoluble in the ester-containing substrate of Formula (I) and/or the alcohol of Formula (II). In preferred processes of the invention the base is substantially insoluble in the solvent.

In preferred processes of the invention, the process further comprises the step of separating the base by filtration. The step of filtering the base after filtration is carried out after the hydrogenation of the ester-containing substrate of Formula (I).

Using a base which is substantially insoluble in the ester-containing substrate of Formula (I) and/or the alcohol of Formula (II) and/or the solvent has the advantage that is can be easily separated from the reaction products, for example from the alcohol of Formula (II). Bases, such as potassium phosphate (K₃PO₄) which are insoluble in organic solvents, such as toluene and THF, therefore offer additional advantages due to their ease of separation by, for example, filtration.

### Solvent

In preferred processes of the invention, the process is carried out in the absence of solvent. This has the advantage of making the process both easier and less expensive to perform.

In alternative preferred processes of the invention, the process is carried out in the presence of a solvent.

Preferably, the solvent is selected from an alcohol, toluene, THF and Me-THF. More preferably, the solvent is selected from toluene, THF and Me-THF. Most preferably, the solvent is selected from toluene and THF.

In preferred processes of the invention, the solvent is present in an amount of 10 to 100 vol% based upon the total volume of the ester-containing substrate, preferably 15 to 95 vol% based upon the total volume of the ester-containing substrate, more preferably 20 to 90 vol% based upon the total volume of the ester-containing substrate (e.g. 50 vol% based upon the total volume of the ester-containing substrate).

In preferred processes of the invention, the volume ratio of the solvent to the ester-containing substrate is less than or equal to 1:1, preferably less than or equal to 1:2.

In preferred processes of the invention, the volume ratio of the solvent to the ester-containing substrate is in the range 1:2 to 1:1, preferably in the range 1:2 to 1:1.5.

In preferred processes of the invention, the process is carried out in the presence of more than one solvent. Preferred solvents are as described above.

In alternative preferred processes of the invention, the process is carried out in the presence of a first solvent and a second solvent.

In preferred processes of the invention, the first solvent is selected from toluene, THF and Me-THF. In preferred processes of the invention, the second solvent is an alcohol, preferably methanol or ethanol.

In particularly preferred processes of the invention, the first solvent is toluene and the second solvent is an alcohol, preferably methanol or ethanol.

In alternative particularly preferred processes of the invention, the first solvent is THF and the second solvent is an alcohol, preferably methanol or ethanol.

In preferred processes of the invention, the first solvent is present in an amount of 10 to 100 vol% based upon the total volume of the ester-containing substrate, preferably 15 to 95 vol% based upon the total volume of the ester-containing substrate, more preferably 20 to 90 vol% based upon the total volume of the ester-containing substrate (e.g. 50 vol% based upon the total volume of the ester-containing substrate).

In preferred processes of the invention, the volume ratio of the first solvent to the ester-containing substrate is less than or equal to 1:1, preferably less than or equal to 1:2.

In preferred processes of the invention, the volume ratio of the first solvent to the ester-containing substrate is in the range 1:2 to 1:1, preferably in the range 1:2 to 1:1.5.

In preferred processes of the invention, the second solvent is present in an amount of 1 to 15 vol% based upon the total volume of ester-containing substrate, preferably in an amount of 1 to 10 vol% based upon the total volume of ester-containing substrate, preferably in an amount of 1 to 7.5 vol% based upon the total volume of the ester-containing substrate, more preferably in an amount of 1 to 5 vol% based upon the total volume of the ester-containing substrate.

In preferred processes of the invention, the first solvent is present in an amount of 10 to 100 vol% based upon the total volume of the ester-containing substrate and the second solvent is present in an amount of 1 to 10 vol% based upon the total volume of ester-containing substrate, preferably the first solvent is present in an amount of 15 to 95 vol% based upon the total volume of the ester-containing substrate and the second solvent is present in an amount of 1 to 7.5 vol% based upon the total volume of the ester-containing substrate, more preferably the first solvent is present in an amount of 20 to 90 vol% based upon the total volume of the ester-containing substrate and the second solvent is present in an amount of 1 to 5 vol% based upon the total volume of the ester-containing substrate.

### By-products

The process of the invention has the surprising advantage of producing lower levels of unwanted by-products.

As described hereinabove, in processes of the prior art and/or where a strong base (e.g. one whose conjugate acid has a pKa of greater than 15, such a metal alkoxide) is used unwanted by-products may be formed.

Unwanted by-products typically comprise wax esters, saturated alcohols, saturated esters, and hemiacetals. Without wishing to be bound by any sort of theory, the formation of these unwanted by-products are believed to result from the formation of enolate intermediate compounds. Accordingly, in the process of the present invention it is believed that lower levels of unwanted by-products are formed because the formation of enolate intermediates is minimised or eliminated.

### Temperature

The process of the invention may be conducted at a temperature in the range 20 to 150 °C. In preferred processes of the invention the process may be conducted at a temperature in the range 40 to 90 °C, more preferably in the range 40 to 85 °C, even more preferably in the range 50 to 85 °C, even more preferably in the range 50 to 80 °C, even more preferably in the range 55 to 75 °C, and most preferably in the range 60 to 75 °C (e.g. about 70 °C).

The preferred processes of the invention are conducted at relatively low temperatures. It has surprisingly been found that using a temperature within the preferred ranges described hereinabove produces fewer undesirable by-products than when higher temperatures are used whilst maintaining a good rate of reaction.

In particularly preferred embodiments of the process the solvent is toluene and the process is conducted at a temperature in the range 40 to 90 °C, more preferably in the range 45 to 85 °C, even more preferably in the range 50 to 85 °C, even more preferably in the range 50 to 80 °C, even more preferably in the range 55 to 75 °C, and most preferably in the range 60 to 75 °C (e.g. about 70 °C).

It is surprising that when toluene is the solvent that temperatures lower than the atmospheric pressure boiling point of toluene (ca. 110 °C) gives improved uptake of hydrogen gas in the hydrogenation process.

### Pressure

Preferred processes of the invention are conducted at a pressure that is at least 5 bar, more preferably at least 10 bar, even more preferably at least 20 bar, even more preferably at least 30 bar, even more preferably at least 40 bar, and most preferably at least 50 bar.

Preferred processes of the invention are conducted at a pressure that is in the range 5 to 100 bar, more preferably in the range 10 to 95 bar, even more preferably in the range 20 to 90 bar, even more preferably in the range 25 to 70 bar, and most preferably in the range 30 to 50 bar.

### Duration

Preferred processes of the invention are conducted for a duration of 1 to 24 hours, more preferably 2 to 20 hours, even more preferably 3 to 18 hours, and most preferably 4 to 16 hours.

### S/C - Substrate to Catalyst

In preferred processes of the invention, the substrate/catalyst loading is greater than or equal to 500/1, preferably greater than or equal to 650/1, even more preferably greater than or equal to 750/1, even more preferably greater than or equal to 850/1. In preferred processes of the invention, the substrate/catalyst loading is less than or equal to 50,000/1, preferably less than or equal to 30,000/1, even more preferably less than or equal to 20,000/1, even more preferably less than or equal to 10,000/1. For example, in preferred processes of the invention, the substrate/catalyst loading is greater than or equal to 500/1 and less than or equal to 50,000/1, preferably the substrate/catalyst loading is greater than or equal to 650/1 and less than or equal to 30,000/1, even more preferably the substrate/catalyst loading is greater than or equal to 750/1 and less than or equal to 20,000/1, even more preferably the substrate/catalyst loading is greater than or equal to 1,000/1 and less than or equal to 10,000/1, for example 5,000/1.

### Transition Metal Catalyst

The process of the invention employs a transition metal catalyst. The transition metal catalyst may be pre-formed or may be formed in situ during the ester hydrogenation reaction. Preferably, the transition metal catalyst is pre-formed. Alternatively, the transition metal catalyst is formed in situ during the ester hydrogenation reaction.

In preferred processes of the invention, the transition metal in the transition metal catalyst is a Group 6, Group 7, Group 8, or Group 9 transition metal. More preferably, the transition metal in the transition metal catalyst is a Group 7, Group 8, or Group 9 transition metal. Even more preferably, the transition metal in the transition metal catalyst is a Group 8 transition metal.

In preferred processes of the invention, the transition metal in the transition metal catalyst is selected from Mo, Mn, Fe, Ru, Co and Os. More preferably, the transition metal in the transition metal catalyst is selected from Ru and Os. Most preferably, the transition metal in the transition metal catalyst is Ru.

In preferred processes of the invention, the transition metal catalyst employed in the process of the invention comprises a tridentate ligand.

In preferred processes of the invention, the transition metal catalyst comprises a tridentate ligand having a Formula (III) 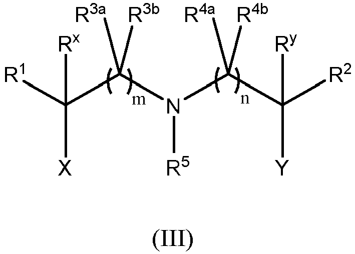 wherein:
X is selected from -SR^{a}, -OR^{a}, -CR^{a}, -NR^{a}R^{b}, -PR^{a}R^{b}, -P(=O)R^{a}R^{b}, -OPR^{a}R^{b}, and - NHPR^{a}R^{b};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R¹ and one of R^{3a} and R^{3b} or R^{x} and one of R^{3a} and R^{3b} together with the atoms to which they are bound, form a ring;
or X is a heteroatom and when taken together with R¹ it forms an optionally substituted heterocycle when R^{x} is absent;
Y is selected from -SR^{a}, -OR^{a}, -CR^{a}, -NR^{a}R^{b}, -PR^{a}R^{b}, -P(=O)R^{a}R^{b}, -OPR^{a}R^{b}, and - NHPR^{a}R^{b};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R² and one of R^{4a} and R^{4b} or R^{y} and one of R^{4a} and R^{4b} together with the atoms to which they are bound, form a ring;
or Y is a heteroatom and when taken together with R² it forms an optionally substituted heterocycle when R^{y} is absent;
R^{3a}_{,} R^{3b}, R^{4a}, and R^{4b} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R^{3a} and one of R^{4a} and R^{4b} or R^{3b} and one of R^{4a} and R^{4b} together with the atoms to which they are bound, form a heterocycle;
R⁵ is selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or when X and/or Y is -NR^{a}R^{b}, -PR^{a}R^{b}, -OPR^{a}R^{b}, or -NHPR^{a}R^{b}, R^{a} and R^{b} together with the heteroatom to which they are attached form a heterocycle.

In tridentate ligands of Formula (III), X is preferably selected from -SR^{a}, -CR^{a}, -NR^{a}R^{b}, - PR^{a}R^{b}, and -NHPR^{a}R^{b}. More preferably, X is selected from -SR^{a}, -PR^{a}R^{b}, and -NHPR^{a}R^{b}. Even more preferably, X is selected from -SR^{a} and -PR^{a}R^{b}. Most preferably, X is -SR^{a}.

In tridentate ligands of Formula (III), R¹ and R^{x} are each independently preferably selected from hydrogen, substituted or unsubstituted C₁₋₂₀-alkyl, substituted or unsubstituted C₁₋₂₀-heteroalkyl and substituted or unsubstituted C₃₋₂₀-cycloalkyl. More preferably, R¹ and R^{x} are each independently selected from hydrogen and substituted or unsubstituted C₁₋₂₀-alkyl. Even more preferably, R¹ and R^{x} are each hydrogen.

In alternative preferred tridentate ligands of Formula (III) R^{x} is absent, X is a heteroatom and when taken together with R¹ it forms an optionally substituted heterocycle. More preferably, R^{x} is absent, X is a heteroatom and when taken together with R¹ it forms an optionally substituted heteroaromatic ring. More preferably, the optionally substituted heteroaromatic ring is an optionally substituted nitrogen-containing heteroaromatic ring. Even more preferably, the optionally substituted nitrogen-containing heteroaromatic ring is selected from pyridinyl, pyrrolyl, imidazolyl, pyrazolyl, thiazolyl, isothiazolyl, oxazolyl, isoxazolyl, triazolyl, tetrazolyl, thiadiazolyl, oxadiazolyl, pyrimidyl, benzoxazolyl, benzthiazolyl, benzimidazolyl, indolyl, and quinolinyl. Even more preferably, the optionally substituted nitrogen-containing heteroaromatic ring is selected from pyridinyl, pyrrolyl, imidazolyl, pyrazolyl, triazolyl, tetrazolyl, and pyrimidyl. Most preferably, the optionally substituted nitrogen-containing heteroaromatic ring is pyridinyl.

In tridentate ligands of Formula (III), Y is preferably selected from -SR^{a}, -CR^{a}, -NR^{a}R^{b}, - PR^{a}R^{b}, and -NHPR^{a}R^{b}. More preferably, Y is selected from -SR^{a}, -PR^{a}R^{b}, and -NHPR^{a}R^{b}. Even more preferably, Y is selected from -SR^{a} and -PR^{a}R^{b}. Most preferably, Y is -SR^{a}.

In tridentate ligands of Formula (III), R² and R^{y} are each independently preferably selected from hydrogen, substituted or unsubstituted C₁₋₂₀-alkyl, substituted or unsubstituted C₁₋₂₀-heteroalkyl and substituted or unsubstituted C₃₋₂₀-cycloalkyl. More preferably, R² and R^{y} are each independently selected from hydrogen and substituted or unsubstituted C₁₋₂₀-alkyl. Even more preferably, R² and R^{y} are each hydrogen.

In alternative preferred tridentate ligands of Formula (III), Y is a heteroatom and when taken together with R² it forms an optionally substituted heterocycle when R^{y} is absent. More preferably, Y is a heteroatom and when taken together with R² it forms an optionally substituted heteroaromatic ring when R^{y} is absent. More preferably, the optionally substituted heteroaromatic ring is an optionally substituted nitrogen-containing heteroaromatic ring. Even more preferably, the optionally substituted nitrogen-containing heteroaromatic ring is selected from pyridinyl, pyrrolyl, imidazolyl, pyrazolyl, thiazolyl, isothiazolyl, oxazolyl, isoxazolyl, triazolyl, tetrazolyl, thiadiazolyl, oxadiazolyl, pyrimidyl, benzoxazolyl, benzthiazolyl, benzimidazolyl, indolyl, and quinolinyl. Even more preferably, the optionally substituted nitrogen-containing heteroaromatic ring is selected from pyridinyl, pyrrolyl, imidazolyl, pyrazolyl, triazolyl, tetrazolyl, and pyrimidyl. Most preferably, the optionally substituted nitrogen-containing heteroaromatic ring is pyridinyl.

In tridentate ligands of Formula (III), R^{3a}, R^{3b}, R^{4a}, and R^{4b} are each independently preferably selected from hydrogen, substituted or unsubstituted C₁₋₂₀-alkyl, substituted or unsubstituted C₁₋₂₀-heteroalkyl and substituted or unsubstituted C₃₋₂₀-cycloalkyl. More preferably, R^{3a}, R^{3b}, R^{4a}, and R^{4b} are each independently selected from hydrogen and substituted or unsubstituted C₁₋₂₀-alkyl. Even more preferably, R^{3a}, R^{3b}, R^{4a}, and R^{4b} are each hydrogen.

In alternative preferred tridentate ligands of Formula (III), R^{3a} and one of R^{4a} and R^{4b} or R^{3b} and one of R^{4a} and R^{4b} together with the atoms to which they are bound, form a heterocycle. Preferably, the heterocycle is a six-membered ring heterocycle.

In tridentate ligands of Formula (III), R⁵ is preferably selected from hydrogen, substituted or unsubstituted C₁₋₂₀-alkyl, substituted or unsubstituted C₁₋₂₀-heteroalkyl and substituted or unsubstituted C₃₋₂₀-cycloalkyl. More preferably, R⁵ is selected from hydrogen and substituted or unsubstituted C₁₋₂₀-alkyl. Even more preferably, R⁵ is hydrogen.

In tridentate ligands of Formula (III), each m and n is preferably 1.

In tridentate ligands of Formula (III), R^{a} and R^{b}, if present, are each independently preferably selected from hydrogen, substituted or unsubstituted C₁₋₂₀-alkyl, substituted or unsubstituted C₁₋₂₀-heteroalkyl, substituted or unsubstituted C₃₋₂₀-cycloalkyl, substituted or unsubstituted C₆₋₂₀-aryl, and substituted or unsubstituted C₄₋₂₀-heteroaryl. More preferably, R^{a} and R^{b}, if present, are each independently selected from hydrogen, substituted or unsubstituted C₁₋₂₀-alkyl (e.g. C₁₋₁₀-alkyl) and substituted or unsubstituted C₆₋₂₀-aryl. Particularly preferred C₁₋₂₀-alkyl groups include ethyl, ethyl, n-propyl, iso-propyl, n-butyl, sec-butyl, tert-butyl, pentyl, and hexyl, more preferably methyl, ethyl, iso-propyl, tert-butyl, even more preferably ethyl. Preferred C₆₋₂₀-aryl groups include phenyl, tolyl, xylyl, and methoxyphenyl, more preferably phenyl.

In alternative preferred tridentate ligands of Formula (III), when X and/or Y is -NR^{a}R^{b}, -PR^{a}R^{b}, -OPR^{a}R^{b}, or -NHPR^{a}R^{b}, R^{a} and R^{b} together with the heteroatom to which they are attached form a heterocycle.

In preferred processes of the invention, the transition metal catalyst comprises a tridentate ligand having a Formula (III)
wherein:
X is selected from -SR^{a}, -CR^{a}, -NR^{a}R^{b}, -PR^{a}R^{b}, and -NHPR^{a}R^{b};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or X is a heteroatom and when taken together with R¹ it forms an optionally substituted heteroaromatic ring when R^{x} is absent, wherein the heteroaromatic ring is a nitrogen-containing heteroaromatic ring;
Y is selected from -SR^{a}, -CR^{a}, -NR^{a}R^{b}, -PR^{a}R^{b}, and -NHPR^{a}R^{b}-
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or Y is a heteroatom and when taken together with R² it forms an optionally substituted heteroaromatic ring when R^{y} is absent, wherein the heteroaromatic ring is a nitrogen-containing heteroaromatic ring;
R^{3a}_{,} R^{3b}, R^{4a}, R^{4b} and R⁵ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or when X and/or Y is -NR^{a}R^{b}, -PR^{a}R^{b} or -NHPR^{a}R^{b}, R^{a} and R^{b} together with the heteroatom to which they are attached form a heterocycle.

In preferred processes of the invention, the transition metal catalyst comprises a tridentate ligand having a Formula (III)
wherein:
X is selected from -SR^{a}, -PR^{a}R^{b}, and -NHPR^{a}R^{b};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or X is a heteroatom and when taken together with R¹ it forms an optionally substituted heteroaromatic ring when R^{x} is absent, wherein the heteroaromatic ring is a nitrogen-containing heteroaromatic ring selected from pyridinyl, pyrrolyl, imidazolyl, pyrazolyl, thiazolyl, isothiazolyl, oxazolyl, isoxazolyl, triazolyl, tetrazolyl, thiadiazolyl, oxadiazolyl, pyrimidyl, benzoxazolyl, benzthiazolyl, benzimidazolyl, indolyl, and quinolinyl;
Y is selected from -SR^{a}, -PR^{a}R^{b}, and -NHPR^{a}R^{b};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or Y is a heteroatom and when taken together with R² it forms an optionally substituted heteroaromatic ring when R^{y} is absent, wherein the heteroaromatic ring is a nitrogen-containing heteroaromatic ring selected from pyridinyl, pyrrolyl, imidazolyl, pyrazolyl, thiazolyl, isothiazolyl, oxazolyl, isoxazolyl, triazolyl, tetrazolyl, thiadiazolyl, oxadiazolyl, pyrimidyl, benzoxazolyl, benzthiazolyl, benzimidazolyl, indolyl, and quinolinyl;
R^{3a}_{,} R^{3b}_{,} R^{4a}_{,} R^{4b} and R⁵ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or
when X and/or Y is -PR^{a}R^{b} or-NHPR^{a}R^{b}, R^{a} and R^{b} together with the heteroatom to which they are attached form a heterocycle.

In preferred processes of the invention, the transition metal catalyst comprises a tridentate ligand having a Formula (III)
wherein:
X is selected from -SR^{a} and -PR^{a}R^{b};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-Cycloalkyl, substituted or unsubstituted C₃₋₂₀-Cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or X is a heteroatom and when taken together with R¹ it forms an optionally substituted heteroaromatic ring when R^{x} is absent, wherein the heteroaromatic ring is a nitrogen-containing heteroaromatic ring selected from pyridinyl, pyrrolyl, imidazolyl, pyrazolyl, triazolyl, tetrazolyl, and pyrimidyl;
Y is selected from -SR^{a} and -PR^{a}R^{b};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or Y is a heteroatom and when taken together with R² it forms an optionally substituted heteroaromatic ring when R^{y} is absent, wherein the heteroaromatic ring is a nitrogen-containing heteroaromatic ring selected from pyridinyl, pyrrolyl, imidazolyl, pyrazolyl, triazolyl, tetrazolyl, and pyrimidyl;
R^{3a}, R^{3b}, R^{4a}, R^{4b} and R⁵ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or when X and/or Y is -PR^{a}R^{b}, R^{a} and R^{b} together with the heteroatom to which they are attached form a heterocycle.

In preferred processes of the invention, the transition metal catalyst comprises a tridentate ligand having a Formula (III), wherein:
X is -SR^{a};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
Y is -SR^{a};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
R^{3a}, R^{3b}, R^{4a}, R^{4b} and R⁵ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl.

Preferably, the transition metal catalyst comprises a tridentate ligand having a Formula (III), wherein:
X is -SR^{a};
R¹ and R^{x} are each independently selected from hydrogen, substituted or unsubstituted C₁₋₂₀-alkyl, substituted or unsubstituted C₁₋₂₀-heteroalkyl, and substituted or unsubstituted C₃₋₂₀-cycloalkyl;
Y is -SR^{a};
R² and R^{y} are each independently selected from hydrogen, substituted or unsubstituted C₁₋₂₀-alkyl, substituted or unsubstituted C₁₋₂₀-heteroalkyl, and substituted or unsubstituted C₃₋₂₀-cycloalkyl;
R^{3a}, R^{3b}, R^{4a}, R^{4b} and R⁵ are each independently selected from hydrogen, substituted or unsubstituted C₁₋₂₀-alkyl, substituted or unsubstituted C₁₋₂₀-heteroalkyl, and substituted or unsubstituted C₃₋₂₀-cycloalkyl;
each m and n is independently 1 or 2; and
R^{a} are each independently selected from hydrogen, substituted or unsubstituted C₁₋₂₀-alkyl, substituted or unsubstituted C₁₋₂₀-heteroalkyl, and substituted or unsubstituted C₃₋₂₀-cycloalkyl.

More preferably, the transition metal catalyst comprises a tridentate ligand having a Formula (III), wherein:
X is -SR^{a};
R¹ and R^{x} are each independently selected from hydrogen and substituted or unsubstituted C₁₋₂₀-alkyl;
Y is -SR^{a};
R² and R^{y} are each independently selected from hydrogen and substituted or unsubstituted C₁₋₂₀-alkyl;
R^{3a}, R^{3b}, R^{4a}, R^{4b} and R⁵ are each independently selected from hydrogen and substituted or unsubstituted C₁₋₂₀-alkyl;
each m and n is independently 1 or 2; and
R^{a} are each independently selected from hydrogen and substituted or unsubstituted C₁₋₂₀-alkyl, substituted or unsubstituted C₁₋₂₀-heteroalkyl, and substituted or unsubstituted C₃₋₂₀-cycloalkyl.

Even more preferably, the transition metal catalyst comprises a tridentate ligand having a Formula (III), wherein:
X and Y are each -SR^{a};
R¹, R^{x}, R², R^{y}, R^{3a}, R^{3b}, R^{4a}, R^{4b} and R⁵ are each hydrogen;
m and n are each 1; and
R^{a} are each independently substituted or unsubstituted C₁₋₂₀-alkyl, preferably C₁₋₁₀ alkyl.

Even more preferably, the transition metal catalyst comprises a tridentate ligand having a Formula (III), wherein:
X and Y are each -SEt;
R¹, R^{x}, R², R^{y}, R^{3a}, R^{3b} , R^{4a}, R^{4b} and R⁵ are each hydrogen; and
m and n are each 1.

In alternative preferred processes of the invention, the transition metal catalyst comprises a tridentate ligand having a Formula (III), wherein:
X is a heteroatom and when taken together with R¹ it forms an optionally substituted heteroaromatic ring when R^{x} is absent;
Y is -PR^{a}R^{b};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
R^{3a}, R^{3b}, R^{4a}, R^{4b} and R⁵ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or R^{a} and R^{b} together with the heteroatom to which they are attached form a heterocycle.

Preferably, the transition metal catalyst comprises a tridentate ligand having a Formula (III), wherein:
X is a nitrogen atom and when taken together with R¹ it forms an optionally substituted heteroaromatic ring when R^{x} is absent, wherein the heteroaromatic ring is a nitrogen-containing heteroaromatic ring;
Y is -PR^{a}R^{b};
R² and R^{y} are each independently is selected from hydrogen, substituted or unsubstituted C₁₋₂₀-alkyl, substituted or unsubstituted C₁₋₂₀-heteroalkyl, and substituted or unsubstituted C₃₋₂₀-cycloalkyl;
R^{3a}, R^{3b}, R^{4a}, R^{4b} and R⁵ are each independently selected from hydrogen, substituted or unsubstituted C₁₋₂₀-alkyl, substituted or unsubstituted C₁₋₂₀-heteroalkyl, substituted or unsubstituted C₃₋₂₀-cycloalkyl;
each m and n is independently 1 or 2; and
R^{a} and R^{b} are each independently selected from substituted or unsubstituted C₁₋₂₀-alkyl, substituted or unsubstituted C₁₋₂₀-heteroalkyl, substituted or unsubstituted C₃₋₂₀-cycloalkyl, substituted or unsubstituted C₆₋₂₀-aryl, and substituted or unsubstituted C₄₋₂₀-heteroaryl.

More preferably, the transition metal catalyst comprises a tridentate ligand having a Formula (III), wherein:
X is a nitrogen atom and when taken together with R¹ it forms an optionally substituted heteroaromatic ring when R^{x} is absent, wherein the heteroaromatic ring is a nitrogen-containing heteroaromatic ring selected from pyridinyl, pyrrolyl, imidazolyl, pyrazolyl, thiazolyl, isothiazolyl, oxazolyl, isoxazolyl, triazolyl, tetrazolyl, thiadiazolyl, oxadiazolyl, pyrimidyl, benzoxazolyl, benzthiazolyl, benzimidazolyl, indolyl, and quinolinyl;
Y is -PR^{a}R^{b};
R², R^{y}, R^{3a}, R^{3b}, R^{4a}, R^{4b} and R⁵ are each hydrogen;
each m and n is 1; and
R^{a} and R^{b} are each independently selected from substituted or unsubstituted C₁₋₂₀-alkyl and substituted or unsubstituted C₆₋₂₀-aryl.

Even more preferably, the transition metal catalyst comprises a tridentate ligand having a Formula (III), wherein:
X is a nitrogen atom and when taken together with R¹ it forms an optionally substituted heteroaromatic ring when R^{x} is absent, wherein the heteroaromatic ring is a nitrogen-containing heteroaromatic ring selected from pyridinyl, pyrrolyl, imidazolyl, pyrazolyl, triazolyl, tetrazolyl, and pyrimidyl;
Y is -PR^{a}R^{b};
R², R^{y}, R^{3a}, R^{3b}, R^{4a}, R^{4b} and R⁵ are each hydrogen;
each m and n is 1; and
R^{a} and R^{b} are each independently selected from methyl, ethyl, n-propyl, iso-propyl, n-butyl, sec-butyl, tert-butyl, pentyl, hexyl, phenyl, tolyl, xylyl, and methoxyphenyl.

Even more preferably, the transition metal catalyst comprises a tridentate ligand having a Formula (III), wherein:
X is a nitrogen atom and when taken together with R¹ it forms an optionally substituted pyridinyl ring when R^{x} is absent;
Y is -PR^{a}R^{b};
R², R^{y}, R^{3a}, R^{3b}, R^{4a}, R^{4b} and R⁵ are each hydrogen;
each m and n is 1; and
R^{a} and R^{b} are each independently selected from methyl, ethyl, iso-propyl, tert-butyl, phenyl, tolyl, xylyl, and methoxyphenyl.

Even more preferably, the transition metal catalyst comprises a tridentate ligand having a Formula (III), wherein:
X is a nitrogen atom and when taken together with R¹ it forms an optionally substituted pyridinyl ring when R^{x} is absent;
Y is -PR^{a}R^{b};
R², R^{y}, R^{3a}, R^{3b}, R^{4a}, R^{4b} and R⁵ are each hydrogen;
each m and n is 1; and
R^{a} and R^{b} are each phenyl.

In preferred processes of the invention, the transition metal catalyst has a Formula (IV) or Formula (V)

[M(L¹)(L²)_{d}] (IV)

[M(L¹)(L²)_{d}]W (V)

wherein:
M is a transition metal;
L¹ is a tridentate ligand as hereinbefore defined;
L² are ligands which may be the same or different;
d is 1, 2 or 3; and
W is a non-coordinated anionic ligand.

In preferred processes of the invention, M is a Group 6, Group 7, Group 8, or Group 9 transition metal. More preferably, M is a Group 7, Group 8, or Group 9 transition metal. Even more preferably, M is a Group 8 transition metal.

In preferred processes of the invention, M is a transition metal selected from Mo, Mn, Fe, Ru, Co and Os. More preferably, M is a transition metal selected from Ru and Os. Most preferably, M is Ru.

In preferred processes of the invention, d is 3.

As will be understood by a skilled person, each L² may be a monodentate ligand or a multidentate ligand, provided the combination of L² ligands is allowed by the rules of valency. In preferred processes of the invention, each L² is a monodentate ligand. Preferably, each L² is independently a neutral monodentate ligand or an anionic monodentate ligand. In preferred processes of the invention, each L² is independently selected from -H, -CO, -CN, -P(R')₃, - As(R')₃, -CR', -OR', -O(C=O)R', -NR'₂, halogen (e.g. -Cl, -Br, -I), and solvent, wherein each R' is independently selected from substituted or unsubstituted alkyl, substituted or unsubstituted alkenyl, substituted or unsubstituted alkynyl, substituted or unsubstituted heteroalkyl, substituted or unsubstituted alkoxy, substituted or unsubstituted cycloalkyl, substituted or unsubstituted heterocycloalkyl, substituted or unsubstituted aryl, and substituted or unsubstituted heteroaryl. Preferably, each L² is independently selected from -H, -CO, -P(R')₃, and halogen. More preferably, each L² is independently selected from -CO, -PPh₃, and -Cl. When L² is solvent, the solvent is preferably selected from THF, Me-THF, MeCN, H₂O and an alcohol (e.g. methanol, ethanol, iso-propanol etc.).

In the transition metal catalysts of Formula (V), W is a non-coordinated anionic ligand. By "non-coordinated anion ligand", we mean the anionic ligand is forced to the outer sphere of the metal centre. The anionic ligand, therefore, is dissociated from the metal centre. This is in contrast to neutral complexes in which the anionic ligand is bound to the metal within the coordination sphere. The anionic ligand can be generally identified as non-coordinating by analysing the X-ray crystal structure of the cationic complex. Preferably, W is selected from the group consisting of triflate (i.e. TfO⁻ or CF₃SO₃⁻), tetrafluoroborate (i.e. ⁻BF₄), hexafluoroantimonate (i.e. ⁻SbF₆), hexafluorophosphate (PF₆⁻), [B[3,5-(CF₃)₂C₆H₃]_{4]}⁻ ([BAr^{F}₄]⁻), halide (e.g. Cl⁻, Br, I⁻) and mesylate (MsO⁻ or MeSO₃⁻).

Preferably, the transition metal catalyst is a transition metal catalyst of Formula (IV).

Alternatively, the transition metal catalyst is a transition metal catalyst of Formula (V).

In preferred processes of the invention, the transition metal catalyst is a transition metal catalyst selected from: 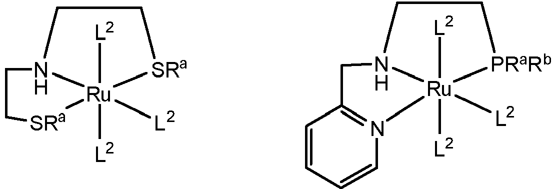 for example: 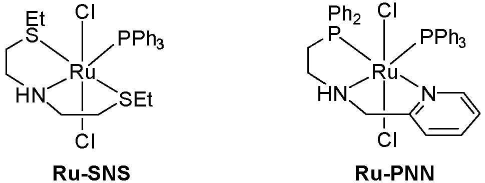 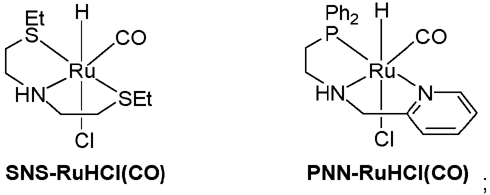 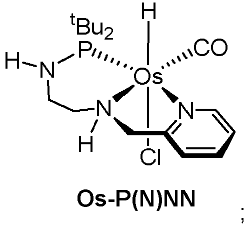 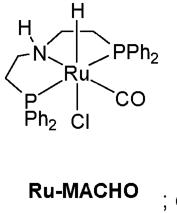 or

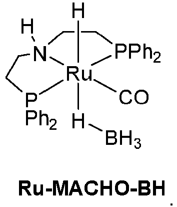

In preferred processes of the invention, the transition metal catalyst is

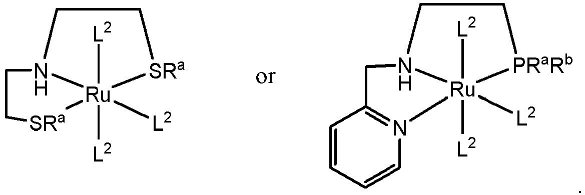

In preferred processes of the invention, the transition metal catalyst is Ru-SNS, Ru-SNN, or Ru-PNN. More preferably, the transition metal catalyst is Ru-SNS or Ru-PNN.

In preferred processes of the invention, the transition metal catalyst is

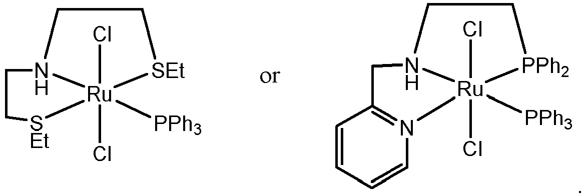

In preferred processes of the invention, the transition metal catalyst employed in the processes of the invention comprises a bidentate ligand.

In preferred processes of the invention, the transition metal catalyst comprises a bidentate ligand having a Formula (VI) 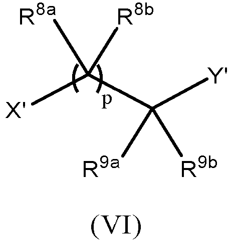 wherein:
X' is -NHR^{ax};
Y' is selected from -SR^{ax}, -OR^{ax} -CR^{ax}, -NR^{ax}R^{bx}, -PR^{ax}R^{bx}, -P(=O)R^{ax}R^{bx}, -OPR^{ax}R^{bx}, and -NHPR^{ax}R^{bx};
R^{8a}, R^{8b}, R^{9a} and R^{9b} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
p is 1 or 2; and
R^{ax} and R^{bx}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or when X' and/or Y' is -NR^{ax}R^{bx}, -PR^{ax}R^{bx}, -OPR^{ax}R^{bx}, or-NHPR^{ax}R^{bx}, R^{ax} and R^{bx} together with the heteroatom to which they are attached form a heterocycle.

In preferred processes of the invention, the transition metal catalyst has a Formula (VII) or Formula (VIII)

[M(L¹)ₑ(L²)_{f}] (VII)

[M(L¹)ₑ(L²)_{f}]W (VIII)

wherein:
M is a transition metal;
L¹ are bidentate ligands as hereinbefore defined which may be the same or different;
L², if present, are ligands which may be the same or different;
e is 1 or 2 such that when e is 1, f is 2, 3 or 4, and when e is 2, f is 0, 1 or 2; and
W is a non-coordinated anionic ligand.
   M, L² and W are as generally described above.

In preferred processes of the invention, the transition metal catalyst employed in the processes of the invention comprises a tetradentate ligand.

In preferred processes of the invention, the transition metal catalyst comprises a tetradentate ligand having a Formula (IX) 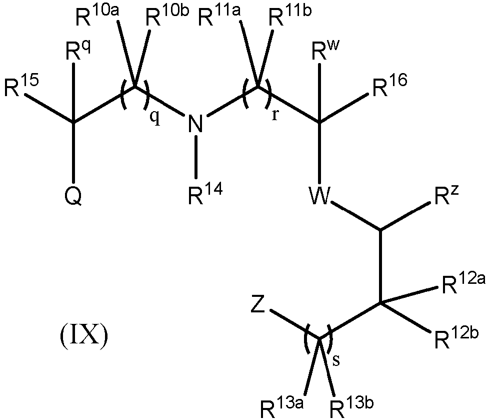 wherein:
Q is selected from -SR^{ay}, -OR^{ay}, -CR^{ay}, -NR^{ay}R^{by}, -PR^{ay}R^{by}, -P(=O)R^{ay}R^{by}, -OPR^{ay}R^{by}, and -NHPR^{ay}R^{by};
R¹⁵ and R^{q} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or Q is a heteroatom and when taken together with R¹⁵ it forms an optionally substituted heterocycle when R^{q} is absent;
W is selected from S, O, NR^{a}, and PR^{a};
R¹⁶ , R^{w} and R^{z} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
or R¹⁶ when taken together with R^{z} forms an optionally substituted heterocycle when R^{w} is absent;
Z is selected from -SR^{ay}, -OR^{ay}, -CR^{ay}, -NR^{ay}R^{by}, -PR^{ay}R^{by}, -P(=O)R^{ay}R^{by}, -OPR^{ay}R^{by}, and - NHPR^{ay}R^{by};
R^{10a}, R^{10b}, R^{11a}, and R^{11b} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or R^{10a} and one of R^{11a} and R^{11b} or R^{10b} and one of R^{11a} and R^{11b} together with the atoms to which they are bound, form a heterocycle;
R^{12a}, R^{12b}, R^{13a}, R^{13b} and R¹⁴ are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each q and r is independently 1 or 2;
s is 0, 1 or 2; and
R^{ay} and R^{by}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or when Q and/or Z is -NR^{ay}R^{by}, -PR^{ay}R^{by}, -OPR^{ay}R^{by}, or-NHPR^{ay}R^{by}, R^{ay} and R^{by} together with the heteroatom to which they are attached form a heterocycle.

In preferred processes of the invention, the transition metal catalyst has a Formula (X) or Formula (XI)

[M(L¹)(L²)_{g}] (X)

[M(L¹)(L²)_{g}]W (XI)

wherein:
M is a transition metal;
L¹ is a tetradentate ligand as hereinbefore defined;
L², if present, are ligands which may be the same or different;
g is 0, 1 or 2; and
W is a non-coordinated anionic ligand.
   M, L² and W are as generally described above.

In preferred processes of the invention, the transition metal catalyst is removed from the reaction mixture by a precipitation step using a co-solvent.

In alternative preferred processes of the invention, the transition metal catalyst is removed from the reaction mixture by distillation of the product.

In alternative preferred processes of the invention, the transition metal catalyst is removed from the reaction mixture by crystallisation of the product.

In alternative preferred processes of the invention, the transition metal catalyst is removed from the reaction mixture using a metal scavenger.

In preferred processes of the invention, the process is a flow process. Preferably, the process is a flow process wherein any excess base is recycled or reused. The base may be recycled or reused once, twice, three times, or more.

The invention will now be further described by way of the following non-limiting examples.

### Examples

Catalysts Ru-SNS, Ru-PNN, SNS-RuHCl(CO), and PNN-RuHCl(CO) are commercially available from Johnson Matthey PLC and were used as supplied.

Ru-MACHO-BH is available from Strem Chemicals and was used as supplied.

OsPNN(N) is available from Merck and was used as supplied.

K₃PO₄ and NaOEt are available from Acros Organics, Alfa Aesar, Fischer Scientific, and Merck. K₃PO₄ was stored inside a glovebox filled with argon gas until use. Liquid reagents and anhydrous solvents were stored under nitrogen and transferred to reaction vials using standard air sensitive handling procedures.

Reactions were carried out in a Biotage Endeavour multi-well pressurised reaction system using 10 mL reaction vials.

### General Testing Procedure

The transition metal catalyst was added to the reaction vials along with the base in an argon filled glovebox. Anhydrous solvent was injected into the reaction vials, followed by the substrate via syringe. The reactions were sealed in the Biotage Endeavour multi-well pressurised reaction system. The reaction vials were purged with nitrogen five times, and purged with hydrogen once before being sealed and heated to the desired reaction temperature. The reaction vessels were pressurised with hydrogen to 2.8 MPa (400 psi) with constant stirring at from 450 to 600 rpm. Each reaction was carried out for a period of 16 hours. The reaction mixtures were analysed by gas chromatography (GC) using ethanol as a solvent.

### Measurement methods

Gas chromatography (GC) measurements were conducted using a Varian 3900 or 3800 gas chromatograph system. Unless otherwise indicated, reaction conversions were determined by GC analysis.

### Example 1: Ester Hydrogenation

The General Procedure described above was carried out on the following ester-containing substrates of Formula (I):

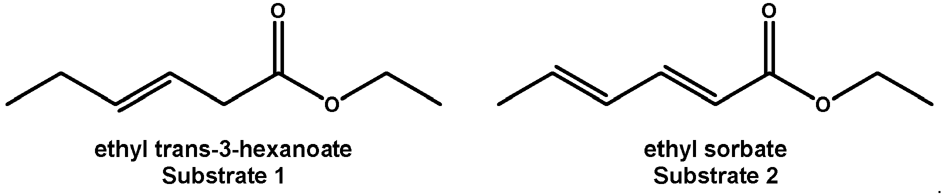

The desired products from Substrate 1 and Substrate 2 were the corresponding alcohols of Formula (II), namely:

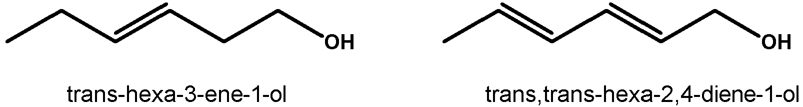

The base was varied between a "weak base" (e.g. K₃PO₄) according to the process of the invention, and a "strong base" not according to the process of the invention (e.g. NaOEt, or NaO^{t}Bu)

Various reactions conditions were varied including the amount of base (relative to the substrate), substrate to transition metal catalyst, reaction temperature, solvent system, and transition metal catalyst. The transition metal catalysts used in the examples are as defined hereinabove. The results, as analysed by gas chromatography, show the conversion to the corresponding alcohol of Formula (II), the molar amount of unreacted starting material, the molar amount of saturated starting material (i.e. the instance where the double bond(s) of the starting material have been reduced but not the ester), and the molar amount of waxy ester by-products. The results of these experiments are summarised in Table 1 below. Examples 1-10 and 17-48 are according to the invention, whilst Examples 11-16 and 49-56 are comparative examples.

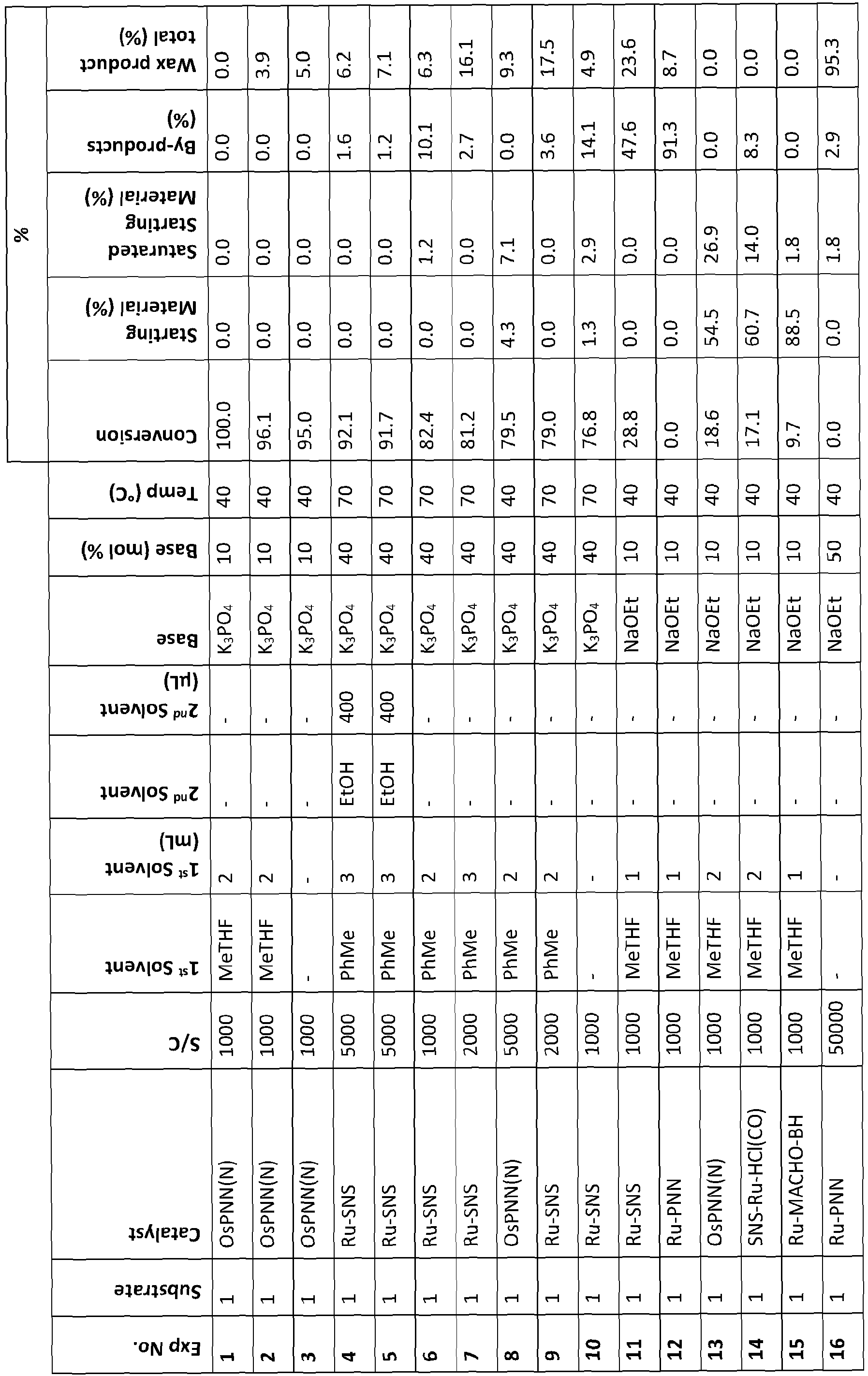

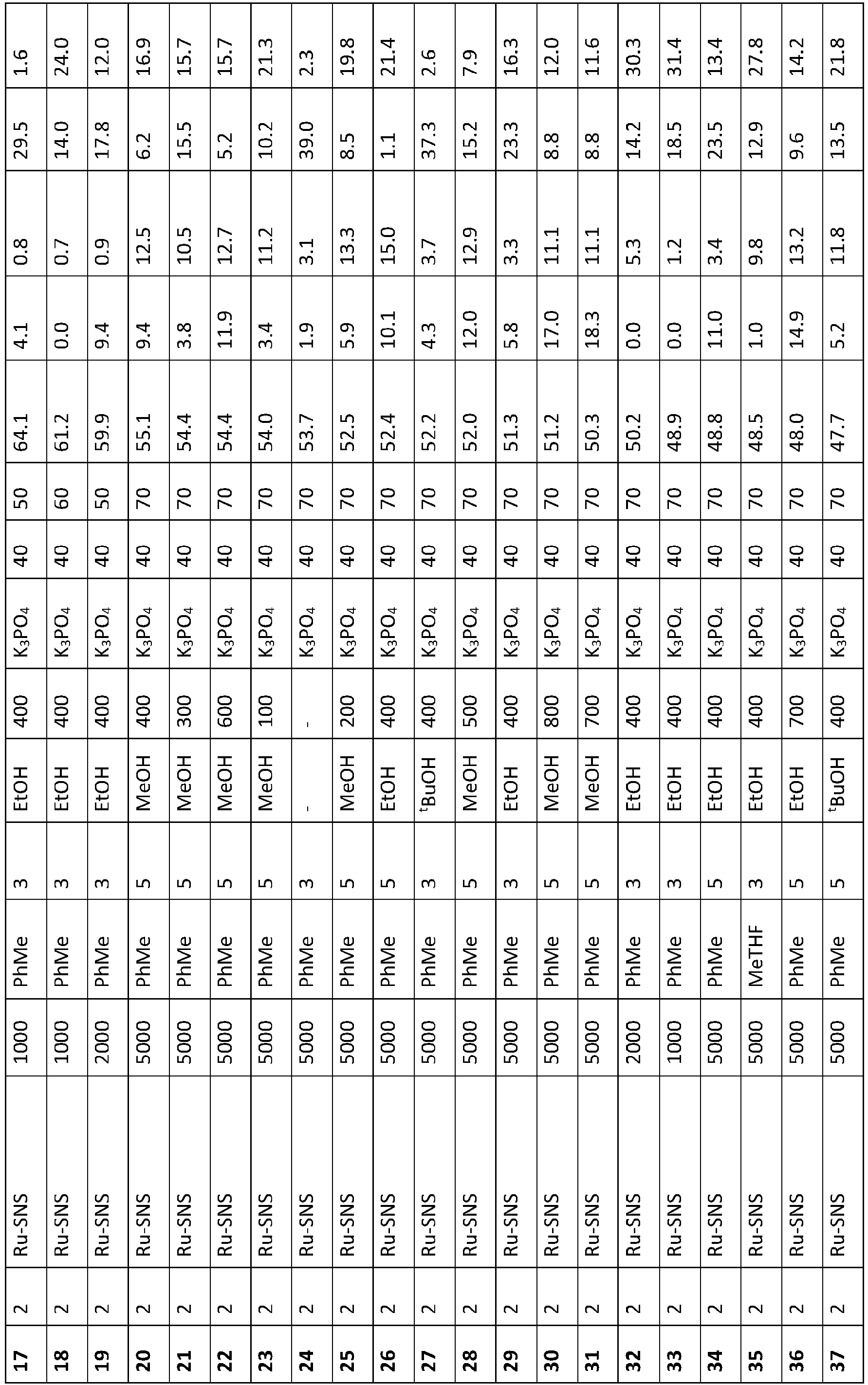

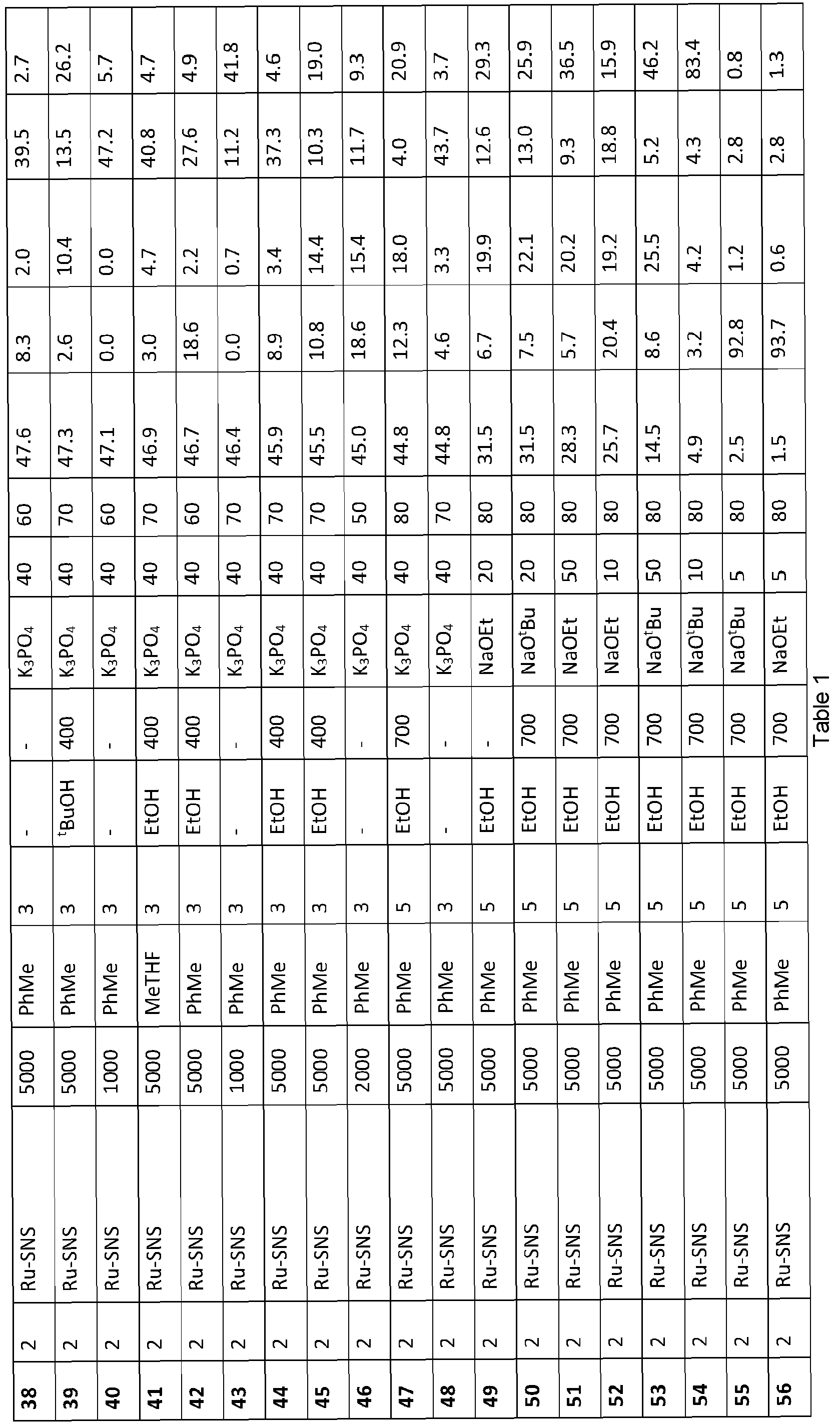

The examples show that excellent conversion to the desired alcohol of Formula (II), with minimal by-product formation, can be achieved by the process of the present invention. In particular, low catalyst loadings can be used in a variety of solvent systems.

In contrast to when a base according to the invention is used, when a 'strong' base, such as NaOEt or Nao^{t}Bu is used, poor conversion the desired alcohol of Formula (II) is achieved. Instead, by-products are formed such as those where the alkene bond(s) have been reduced, those comprising waxy ester materials, or other unidentified by-products.

## Claims

1. A process for the hydrogenation of an ester-containing substrate of Formula (I) to produce an alcohol of Formula (II), 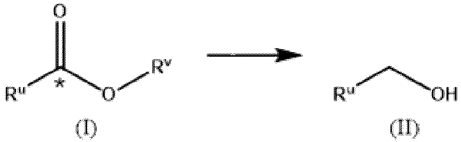 the process comprising treating the ester-containing substrate of Formula (I) with a base and a transition metal catalyst in the presence of molecular hydrogen, wherein:
the ester-containing substrate of Formula (I) comprises an α,β-γ,δ unsaturated ester or a β,γ unsaturated ester;
R^{u} is an organic group having 3-70 carbon atoms, having the proviso that R^{u} is bonded to the carbonyl carbon (*) of the ester to form the α,β-γ,δ unsaturated ester or the β,γ unsaturated ester of Formula (I);
R^{v} is an organic group having 1-70 carbon atoms; and
the conjugate acid of the base has a pKa of from 4 to 15.

2. A process according to claim 1, wherein R^{u} is an organic group having Formula (XII): 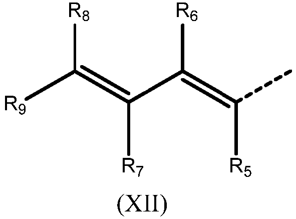
wherein the dashed bond indicates the bond to the carbonyl carbon (*) of the ester moiety of the ester-containing substrate of Formula (I); and
R₅-R₈ are each independently a hydrogen atom or an organic group having 1-70 carbon atoms, and
R₉ is H or an organic group having 1-70 carbon atoms.

3. A process according to claim 1, wherein R^{u} is an organic group having Formula (XIII): 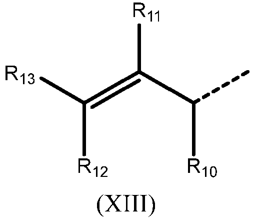
wherein the dashed bond indicates the bond to the carbonyl carbon (*) of the ester moiety of the ester-containing substrate of Formula (I); and
R₁₀-R₁₂ are each independently a hydrogen atom or an organic group having 1-70 carbon atoms, and
R₁₃ is H or an organic group having 1-70 carbon atoms.

4. A process according to claim 1, wherein R^{u} is an organic group having Formula (XII) or an organic group having formula (XIII) as defined in claim 2 or claim 3.

5. A process according to claim 1, wherein the ester-containing substrate of Formula (I) is an alkyl sorbate or an alkyl trans-3-hexanoate

6. A process according to any one of the preceding claims wherein the alcohol of Formula (II) is trans,trans-hexa-2,4-diene-1-ol, cis- hexa-3-ene-1-ol, or trans-hexa-3-ene-1-ol.

7. A process according to any one of the preceding claims wherein the base is a metal phosphate or a metal carbonate, preferably an alkali metal phosphate, an alkali earth metal phosphate, an alkali metal carbonate, or an alkali earth metal carbonate.

8. A process according to any one of the preceding claims, wherein the base is present in solid form.

9. A process according to any one of the preceding claims, wherein the base is present in a range of 30 to 70 mol% based upon the total amount of ester-containing substrate, in a range of 30 to 60 mol% based upon the total amount of ester-containing substrate, or in a range of 30 to 50 mol% based upon the total amount of ester-containing substrate.

10. A process according to any one of the preceding claims, wherein the transition metal catalyst comprises a tridentate ligand.

11. A process according to claim 10, wherein the transition metal catalyst comprises a tridentate ligand having a Formula (III) 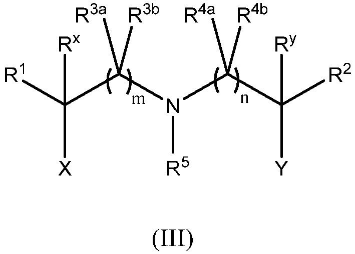 wherein:
X is selected from -SR^{a}, -OR^{a}, -CR^{a}, -NR^{a}R^{b}, -PR^{a}R^{b}, -P(=O)R^{a}R^{b}, -OPR^{a}R^{b}, and - NHPR^{a}R^{b};
R¹ and R^{x} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R¹ and one of R^{3a} and R^{3b} or R^{x} and one of R^{3a} and R^{3b} together with the atoms to which they are bound, form a ring;
or X is a heteroatom and when taken together with R¹ it forms an optionally substituted heterocycle when R^{x} is absent;
Y is selected from -SR^{a}, -OR^{a}, -CR^{a}, -NR^{a}R^{b}, -PR^{a}R^{b}, -P(=O)R^{a}R^{b}, -OPR^{a}R^{b}, and - NHPR^{a}R^{b};
R² and R^{y} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R² and one of R^{4a} and R^{4b} or R^{y} and one of R^{4a} and R^{4b} together with the atoms to which they are bound, form a ring;
or Y is a heteroatom and when taken together with R² it forms an optionally substituted heterocycle when R^{y} is absent;
R^{3a}, R^{3b}, R^{4a}, and R^{4b} are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl, or R^{3a} and one of R^{4a} and R^{4b} or R^{3b} and one of R^{4a} and R^{4b} together with the atoms to which they are bound, form a heterocycle;
R⁵ is selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl;
each m and n is independently 1 or 2; and
R^{a} and R^{b}, if present, are each independently selected from hydrogen, substituted or unsubstituted C₁₋₂₀-alkyl, substituted or unsubstituted C₂₋₂₀-alkenyl, substituted or unsubstituted C₂₋₂₀-alkynyl, substituted or unsubstituted C₁₋₂₀-heteroalkyl, substituted or unsubstituted C₁₋₂₀-alkoxy, substituted or unsubstituted C₃₋₂₀-cycloalkyl, substituted or unsubstituted C₃₋₂₀-cycloalkenyl, substituted or unsubstituted C₂₋₂₀-heterocycloalkyl, substituted or unsubstituted C₆₋₂₀-aryl, and substituted or unsubstituted C₄₋₂₀-heteroaryl; or
when X and/or Y is -NR^{a}R^{b}, -PR^{a}R^{b}, -OPR^{a}R^{b}, or -NHPR^{a}R^{b}, R^{a} and R^{b} together with the heteroatom to which they are attached form a heterocycle

12. A process according to any one of the preceding claims, wherein the transition metal catalyst has a Formula (IV) or Formula (V)
[M(L¹)(L²)_{d}] (IV)
[M(L¹)(L²)_{d}]W (V)
wherein:
M is a transition metal;
L¹ is a tridentate ligand as defined in claim 14;
L² are ligands which may be the same or different;
d is 1, 2 or 3; and
W is a non-coordinated anionic ligand.

13. A process according to claim 12, wherein M is a transition metal selected from Ru and Os, preferably Ru.

14. A process according to claim 12 or 13, wherein each L² is independently selected from - H, -CO, -CN, -P(R')₃, -As(R')₃, -CR', -OR', -O(C=O)R', -NR'₂, halogen (e.g. -Cl, -Br, -I), and solvent wherein each R' is independently selected from substituted or unsubstituted alkyl, substituted or unsubstituted alkenyl, substituted or unsubstituted alkynyl, substituted or unsubstituted heteroalkyl, substituted or unsubstituted alkoxy, substituted or unsubstituted cycloalkyl, substituted or unsubstituted heterocycloalkyl, substituted or unsubstituted aryl, and substituted or unsubstituted heteroaryl.

15. A process according to any one of the preceding claims, wherein the transition metal catalyst is

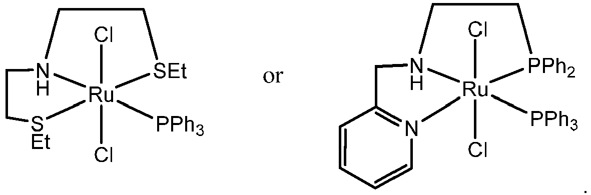

## Patentansprüche

1. Verfahren für die Hydrierung eines esterhaltigen Substrats der Formel (I), um einen Alkohol der Formel (II) herzustellen, 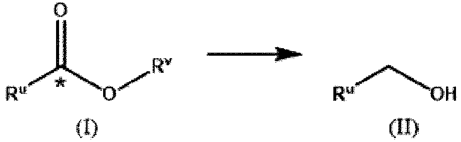 das Verfahren umfassend ein Behandeln des esterhaltigen Substrats der Formel (I) mit einer Base und einem Übergangsmetallkatalysator in der Gegenwart von molekularem Wasserstoff, wobei:
das esterhaltige Substrat der Formel (I) einen α, β-γ, δ-ungesättigten Ester oder einen β, γ-ungesättigten Ester umfasst;
R^{u} eine organische Gruppe ist, die 3-70 Kohlenstoffatome aufweist, mit der Maßgabe, dass R^{u} an den Carbonylkohlenstoff (*) des Esters gebunden ist, um den α, β-γ, δ-ungesättigten Ester oder den β,γ-ungesättigten Ester der Formel (I) auszubilden;
R^{v} eine organische Gruppe ist, die 1-70 Kohlenstoffatome aufweist; und
die konjugierte Säure der Base einen pKa-Wert von 4 bis 15 aufweist.

2. Verfahren nach Anspruch 1, wobei R^{u} eine organische Gruppe ist, die die Formel (XII) aufweist: 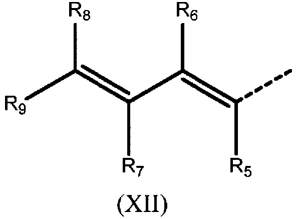
wobei die gestrichelte Bindung die Bindung zu dem Carbonylkohlenstoff (*) des Esterrests des esterhaltigen Substrats der Formel (I) anzeigt; und
R₅-R₈ jeweils unabhängig ein Wasserstoffatom oder eine organische Gruppe sind, die 1-70 Kohlenstoffatome aufweist, und
R₉ H oder eine organische Gruppe ist, die 1-70 Kohlenstoffatome aufweist.

3. Verfahren nach Anspruch 1, wobei R^{u} eine organische Gruppe ist, die die Formel (XIII) aufweist: 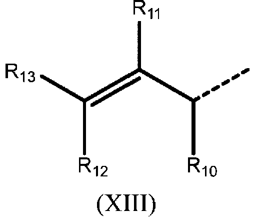
wobei die gestrichelte Bindung die Bindung zu dem Carbonylkohlenstoff (*) des Esterrests des esterhaltigen Substrats der Formel (I) anzeigt; und
R₁₀-R₁₂ jeweils unabhängig ein Wasserstoffatom oder eine organische Gruppe sind, die 1-70 Kohlenstoffatome aufweist, und
R₁₃ H oder eine organische Gruppe ist, die 1-70 Kohlenstoffatome aufweist.

4. Verfahren nach Anspruch 1, wobei R^{u} eine organische Gruppe, die die Formel (XII) aufweist, oder eine organische Gruppe ist, die die Formel (XIII) aufweist, wie in Anspruch 2 oder Anspruch 3 definiert.

5. Verfahren nach Anspruch 1, wobei das esterhaltige Substrat der Formel (I) ein Alkylsorbat oder ein Alkyltrans-3-hexanoat ist.

6. Verfahren nach einem der vorstehenden Ansprüche, wobei der Alkohol der Formel (II) trans,trans-Hexa-2,4-dien-1-ol, cis-Hex-3-en-1-ol oder trans-Hex-3-en-1-ol ist.

7. Verfahren nach einem der vorstehenden Ansprüche, wobei die Base ein Metallphosphat oder ein Metallcarbonat, vorzugsweise ein Alkalimetallphosphat, ein Erdalkalimetallphosphat, ein Alkalimetallcarbonat oder ein Erdalkalimetallcarbonat ist.

8. Verfahren nach einem der vorstehenden Ansprüche, wobei die Base in fester Form vorliegt.

9. Verfahren nach einem der vorstehenden Ansprüche, wobei die Base in einem Bereich von 30 bis 70 mol% bezogen auf die Gesamtmenge an esterhaltigem Substrat, in einem Bereich von 30 bis 60 mol% bezogen auf die Gesamtmenge an esterhaltigem Substrat oder in einem Bereich von 30 bis 50 mol% bezogen auf die Gesamtmenge an esterhaltigem Substrat vorliegt.

10. Verfahren nach einem der vorstehenden Ansprüche, wobei der Übergangsmetallkatalysator einen dreizähnigen Liganden umfasst.

11. Verfahren nach Anspruch 10, wobei der Übergangsmetallkatalysator einen dreizähnigen Liganden umfasst, der eine Formel (III) aufweist 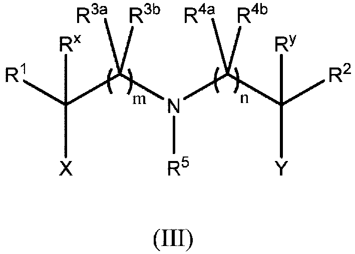 wobei:
X ausgewählt ist aus -SR^{a}, -OR^{a}, -CR^{a}, -NR^{a}R^{b}, -PR^{a}R^{b}, -P(=O)R^{a}R^{b}, -OPR^{a}R^{b} und -NHPR^{a}R^{b};
R¹ und R^{x} jeweils unabhängig ausgewählt sind aus Wasserstoff, substituiertem oder nicht substituiertem C₁₋₂₀-Alkyl, substituiertem oder nicht substituiertem C₂₋₂₀-Alkenyl, substituiertem oder nicht substituiertem C₂₋₂₀-Alkinyl, substituiertem oder nicht substituiertem C₁₋₂₀-Heteroalkyl, substituiertem oder nicht substituiertem C₁₋₂₀-Alkoxy, substituiertem oder nicht substituiertem C₃₋₂₀-Cycloalkyl, substituiertem oder nicht substituiertem C₃₋₂₀-Cycloalkenyl, substituiertem oder nicht substituiertem C₂₋₂₀-Heterocycloalkyl, substituiertem oder nicht substituiertem C₆₋₂₀-Aryl und substituiertem oder nicht substituiertem C₄₋₂₀-Heteroaryl, oder R¹ und eines von R^{3a} und R^{3b} oder R^{x} und eines von R^{3a} und R^{3b} zusammen mit den Atomen, an die sie gebunden sind, einen Ring ausbilden;
oder X ein Heteroatom ist und zusammen mit R¹ einen gegebenenfalls substituierten Heterozyklus ausbildet, wenn R^{x} fehlt;
Y ausgewählt ist aus -SR^{a}, -OR^{a}, -CR^{a}, -NR^{a}R^{b}, -PR^{a}R^{b}, -P(=O)R^{a}R^{b}, -OPR^{a}R^{b} und -NHPR^{a}R^{b};
R² und R^{y} jeweils unabhängig ausgewählt sind aus Wasserstoff, substituiertem oder nicht substituiertem C₁₋₂₀-Alkyl, substituiertem oder nicht substituiertem C₂₋₂₀-Alkenyl, substituiertem oder nicht substituiertem C₂₋₂₀-Alkinyl, substituiertem oder nicht substituiertem C₁₋₂₀-Heteroalkyl, substituiertem oder nicht substituiertem C₁₋₂₀-Alkoxy, substituiertem oder nicht substituiertem C₃₋₂₀-Cycloalkyl, substituiertem oder nicht substituiertem C₃₋₂₀-Cycloalkenyl, substituiertem oder nicht substituiertem C₂₋₂₀-Heterocycloalkyl, substituiertem oder nicht substituiertem C₆₋₂₀-Aryl und substituiertem oder nicht substituiertem C₄₋₂₀-Heteroaryl, oder R² und eines von R^{4a} und R^{4b} oder R^{y} und eines von R^{4a} und R^{4b} zusammen mit den Atomen, an die sie gebunden sind, einen Ring ausbilden;
oder Y ein Heteroatom ist und zusammen mit R² einen gegebenenfalls substituierten Heterozyklus ausbildet, wenn R^{y} fehlt;
R^{3a}, R^{3b}, R^{4a} und R^{4b} jeweils unabhängig ausgewählt sind aus Wasserstoff, substituiertem oder nicht substituiertem C₁₋₂₀-Alkyl, substituiertem oder nicht substituiertem C₂₋₂₀-Alkenyl, substituiertem oder nicht substituiertem C₂₋₂₀-Alkinyl, substituiertem oder nicht substituiertem C₁₋₂₀-Heteroalkyl, substituiertem oder nicht substituiertem C₁₋₂₀-Alkoxy, substituiertem oder nicht substituiertem C₃₋₂₀-Cycloalkyl, substituiertem oder nicht substituiertem C₃₋₂₀-Cycloalkenyl, substituiertem oder nicht substituiertem C₂₋₂₀-Heterocycloalkyl, substituiertem oder nicht substituiertem C₆₋₂₀-Aryl und substituiertem oder nicht substituiertem C₄₋₂₀-Heteroaryl, oder R^{3a} und eines von R^{4a} und R^{4b} ode R^{3b} und eines von R^{4a} und R^{4b} zusammen mit den Atomen, an die sie gebunden sind, einen Heterozyklus ausbilden;
R⁵ ausgewählt ist aus Wasserstoff, substituiertem oder nicht substituiertem C₁₋₂₀-Alkyl, substituiertem oder nicht substituiertem C₂₋₂₀-Alkenyl, substituiertem oder nicht substituiertem C₂₋₂₀-Alkinyl, substituiertem oder nicht substituiertem C₁₋₂₀-Heteroalkyl, substituiertem oder nicht substituiertem C₁₋₂₀-Alkoxy, substituiertem oder nicht substituiertem C₃₋₂₀-Cycloalkyl, substituiertem oder nicht substituiertem C₃₋₂₀-Cycloalkenyl, substituiertem oder nicht substituiertem C₂₋₂₀-Heterocycloalkyl, substituiertem oder nicht substituiertem C₆₋₂₀-Aryl und substituiertem oder nicht substituiertem C₄₋₂₀-Heteroaryl;
jedes m und n unabhängig 1 oder 2 ist; und
R^{a} und R^{b}, falls vorhanden, jeweils unabhängig ausgewählt sind aus Wasserstoff, substituiertem oder nicht substituiertem C₁₋₂₀-Alkyl, substituiertem oder nicht substituiertem C₂₋₂₀-Alkenyl, substituiertem oder nicht substituiertem C₂₋₂₀-Alkinyl, substituiertem oder nicht substituiertem C₁₋₂₀-Heteroalkyl, substituiertem oder nicht substituiertem C₁₋₂₀-Alkoxy, substituiertem oder nicht substituiertem C₃₋₂₀-Cycloalkyl, substituiertem oder nicht substituiertem C₃₋₂₀-Cycloalkenyl, substituiertem oder nicht substituiertem C₂₋₂₀-Heterocycloalkyl, substituiertem oder nicht substituiertem C₆₋₂₀-Aryl und substituiertem oder nicht substituiertem C₄₋₂₀-Heteroaryl; oder wenn X und/oder Y -NR^{a}R^{b}, -PR^{a}R^{b}, -OPR^{a}R^{b} oder -NHPR^{a}R^{b} ist, R^{a} und R^{b} zusammen mit dem Heteroatom, an das sie anhängen, einen Heterozyklus ausbilden.

12. Verfahren nach einem der vorstehenden Ansprüche, wobei der Übergangsmetallkatalysator eine Formel (IV) oder Formel (V) aufweist
[M(L¹)(L²)_{d}] (IV)
[M(L¹)(L²)_{d}]W (V)
wobei:
M ein Übergangsmetall ist;
L¹ ein dreizähniger Ligand wie in Anspruch 14 definiert ist;
L² Liganden sind, die gleich oder verschieden sein können;
d 1, 2 oder 3 ist; und
W ein nicht koordinierender anionischer Ligand ist.

13. Verfahren nach Anspruch 12, wobei M ein Übergangsmetall ist, das aus Ru und Os, vorzugsweise Ru, ausgewählt ist.

14. Verfahren nach Anspruch 12 oder 13, wobei jedes L² unabhängig ausgewählt ist aus - H, -CO, -CN, -P(R')₃, -As(R')₃, -CR', -OR', -O(C=O)R', -NR'₂, Halogen (z.B. -Cl, -Br, -I), und Lösungsmittel, wobei jedes R' unabhängig ausgewählt ist aus substituiertem oder nicht substituiertem Alkyl, substituiertem oder nicht substituiertem Alkenyl, substituiertem oder nicht substituiertem Alkinyl, substituiertem oder nicht substituiertem Heteroalkyl, substituiertem oder nicht substituiertem Alkoxy, substituiertem oder nicht substituiertem Cycloalkyl, substituiertem oder nicht substituiertem Heterocycloalkyl, substituiertem oder nicht substituiertem Aryl, und substituiertem oder nicht substituiertem Heteroaryl.

15. Verfahren nach einem der vorstehenden Ansprüche, wobei der Übergangsmetallkatalysator ist

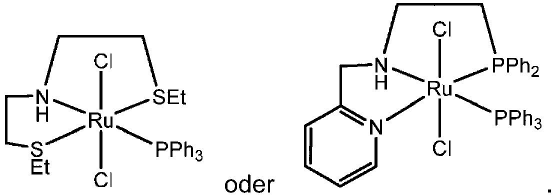

## Revendications

1. Procédé destiné à l'hydrogénation d'un substrat contenant un ester de Formule (I) pour produire un alcool de Formule (II), 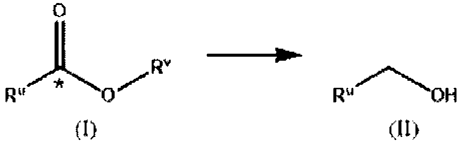 le procédé comprenant le traitement du substrat contenant un ester de Formule (I) avec une base et un catalyseur à métal de transition en présence d'hydrogène moléculaire, dans lequel :
le substrat contenant un ester de Formule (I) comprend un ester à insaturation α, β-γ, δ ou un ester à insaturation β,γ ;
R^{u} est un groupe organique ayant 3 à 70 atomes de carbone, à condition que R^{u} soit lié au carbone (*) de carbonyle de l'ester pour former l'ester à insaturation α, β-γ, δ ou l'ester à insaturation β,γ de Formule (I) ;
R^{v} est un groupe organique ayant 1 à 70 atomes de carbone ; et
l'acide conjugué de la base a un pKa allant de 4 à 15.

2. Procédé selon la revendication 1, dans lequel R^{u} est un groupe organique ayant la Formule (XII) : 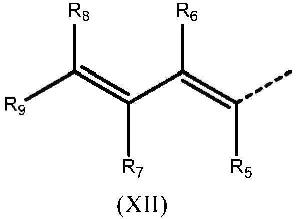
dans lequel la liaison en pointillés indique la liaison au carbone (*) de carbonyle du fragment ester du substrat contenant un ester de Formule (I) ; et
R₅ à R₈ sont chacun indépendamment un atome d'hydrogène ou un groupe organique ayant 1 à 70 atomes de carbone, et
R₉ est H ou un groupe organique ayant 1 à 70 atomes de carbone.

3. Procédé selon la revendication 1, dans lequel R^{u} est un groupe organique ayant la Formule (XIII) : 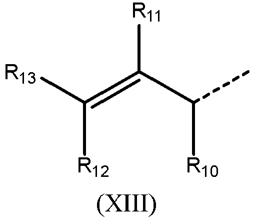
dans lequel la liaison en pointillés indique la liaison au carbone (*) de carbonyle du fragment ester du substrat contenant un ester de Formule (I) ; et
R₁₀ à R₁₂ sont chacun indépendamment un atome d'hydrogène ou un groupe organique ayant 1 à 70 atomes de carbone, et
R₁₃ est H ou un groupe organique ayant 1 à 70 atomes de carbone.

4. Procédé selon la revendication 1, dans lequel R^{u} est un groupe organique ayant la Formule (XII) ou un groupe organique ayant la formule (XIII) tel que défini dans la revendication 2 ou la revendication 3.

5. Procédé selon la revendication 1, dans lequel le substrat contenant un ester de Formule (I) est un sorbate d'alkyle ou un trans-3-hexanoate d'alkyle

6. Procédé selon l'une quelconque des revendications précédentes dans lequel l'alcool de Formule (II) est trans,trans-hexa-2,4-diène-1-ol, cis-hexa-3-ène-1-ol, ou trans-hexa-3-ène-1-ol.

7. Procédé selon l'une quelconque des revendications précédentes dans lequel la base est un phosphate de métal ou un carbonate de métal, de préférence un phosphate de métal alcalin, un phosphate de métal alcalino-terreux, un carbonate de métal alcalin, ou un carbonate de métal alcalino-terreux.

8. Procédé selon l'une quelconque des revendications précédentes, dans lequel la base est présente sous forme solide.

9. Procédé selon l'une quelconque des revendications précédentes, dans lequel la base est présente dans une plage de 30 à 70 % molaires en fonction de la quantité totale de substrat contenant un ester, dans une plage de 30 à 60 % molaires en fonction de la quantité totale de substrat contenant un ester, ou dans une plage de 30 à 50 % molaires en fonction de la quantité totale de substrat contenant un ester.

10. Procédé selon l'une quelconque des revendications précédentes, dans lequel le catalyseur à métal de transition comprend un ligand tridenté.

11. Procédé selon la revendication 10, dans lequel le catalyseur à métal de transition comprend un ligand tridenté ayant une Formule (III) 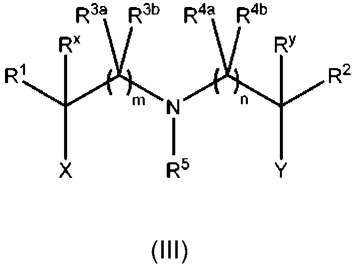 dans lequel :
X est choisi parmi -SR^{a}, -OR^{a}, -CR^{a}, -NR^{a}R^{b}, -PR^{a}R^{b}, -P(=O)R^{a}R^{b}, - OPR^{a}R^{b} et - NHPR^{a}R^{b} ;
R¹ et R^{x} sont chacun indépendamment choisis parmi hydrogène, alkyle en C₁ à ₂₀ substitué ou non substitué, alcényle en C₂ à ₂₀ substitué ou non substitué, alcynyle en C_{2 à 20} substitué ou non substitué, hétéroalkyle en C_{1 à 20} substitué ou non substitué, alcoxy en C_{1 à 20} substitué ou non substitué, cycloalkyle en C_{3 à 20} substitué ou non substitué, cycloalcényle en C_{3 à 20} substitué ou non substitué, hétérocycloalkyle en C_{2 à 20} substitué ou non substitué, aryle en C_{6 à 20} substitué ou non substitué et hétéroaryle en C_{4 à 20} substitué ou non substitué, ou R¹ et l'un de R^{3a} et de R^{3b} ou R^{x} et l'un de R^{3a} et de R^{3b} conjointement avec les atomes auxquels ils sont liés, forment un cycle ;
ou X est un hétéroatome et lorsqu'il est pris ensemble avec R¹ il forme un hétérocycle éventuellement substitué lorsque R^{x} est absent ;
Y est choisi parmi -SR^{a}, -OR^{a}, -CR^{a}, -NR^{a}R^{b}, -PR^{a}R^{b}, -P(=O)R^{a}R^{b}, - OPR^{a}R^{b} et - NHPR^{a}R^{b} ;
R² et R^{y} sont chacun indépendamment choisis parmi hydrogène, alkyle en C_{1 à 20} substitué ou non substitué, alcényle en C_{2 à 20} substitué ou non substitué, alcynyle en C_{2 à 20} substitué ou non substitué, hétéroalkyle en C_{1 à 20} substitué ou non substitué, alcoxy en C_{1 à 20} substitué ou non substitué, cycloalkyle en C_{3 à 20} substitué ou non substitué, cycloalcényle en C_{3 à 20} substitué ou non substitué, hétérocycloalkyle en C_{2 à 20} substitué ou non substitué, aryle en C_{6 à 20} substitué ou non substitué et hétéroaryle en C_{4 à 20} substitué ou non substitué, ou R² et l'un de R^{4a} et de R^{4b}ou R^{y} et l'un de R^{4a} et de R^{4b} conjointement avec les atomes auxquels ils sont liés, forment un cycle ;
ou Y est un hétéroatome et lorsqu'il est pris ensemble avec R² il forme un hétérocycle éventuellement substitué lorsque R^{y} est absent ;
R^{3a}, R^{3b}, R^{4a} et R^{4b} sont chacun indépendamment choisis parmi hydrogène, alkyle en C_{1 à 20} substitué ou non substitué, alcényle en C_{2 à 20} substitué ou non substitué, alcynyle en C_{2 à 20} substitué ou non substitué, hétéroalkyle en C_{1 à 20} substitué ou non substitué, alcoxy en C_{1 à 20} substitué ou non substitué, cycloalkyle en C_{3 à 20} substitué ou non substitué, cycloalcényle en C_{3 à 20} substitué ou non substitué, hétérocycloalkyle en C_{2 à 20} substitué ou non substitué, aryle en C_{6 à 20} substitué ou non substitué et hétéroaryle en C_{4 à 20} substitué ou non substitué, ou R^{3a} et l'un de R^{4a} et de R^{4b}ou R^{3b} et l'un de R^{4a} et de R^{4b} conjointement avec les atomes auxquels ils sont liés, forment un cycle ;
R⁵ est choisi parmi hydrogène, alkyle en C_{1 à 20} substitué ou non substitué, alcényle en C_{2 à 20} substitué ou non substitué, alcynyle en C_{2 à 20} substitué ou non substitué, hétéroalkyle en C_{1 à 20} substitué ou non substitué, alcoxy en C_{1 à 20} substitué ou non substitué, cycloalkyle en C_{3 à 20} substitué ou non substitué, cycloalcényle en C_{3 à 20} substitué ou non substitué, hétérocycloalkyle en C_{2 à 20} substitué ou non substitué, aryle en C_{6 à 20} substitué ou non substitué, et hétéroaryle en C_{4 à 20} substitué ou non substitué ;
chacun de m et n est indépendamment 1 ou 2 ; et
R^{a} et R^{b}, s'ils sont présents, sont chacun indépendamment choisis parmi hydrogène, alkyle en C_{1 à 20} substitué ou non substitué, alcényle en C_{2 à 20} substitué ou non substitué, alcynyle en C_{2 à 20} substitué ou non substitué, hétéroalkyle en C_{1 à 20} substitué ou non substitué, alcoxy en C_{1 à 20} substitué ou non substitué, cycloalkyle en C_{3 à 20} substitué ou non substitué, cycloalcényle en C_{3 à 20} substitué ou non substitué, hétérocycloalkyle en C_{2 à 20} substitué ou non substitué, aryle en C_{6 à 20} substitué ou non substitué, et hétéroaryle en C_{4 à 20} substitué ou non substitué ; ou lorsque X et/ou Y sont -NR^{a}R^{b}, -PR^{a}R^{b}, -OPR^{a}R^{b}, ou -NHPR^{a}R^{b}, R^{a} et R^{b} conjointement avec l'hétéroatome auquel ils sont fixés forment un hétérocycle

12. Procédé selon l'une quelconque des revendications précédentes, dans lequel le catalyseur à métal de transition a une Formule (IV) ou une Formule (V)
[M(L¹)(L²)_{d}] (IV)
[M(L¹)(L²)_{d}]W (V)
dans lequel :
M est un métal de transition ;
L¹ est un ligand tridenté tel que défini dans la revendication 14 ;
L² sont des ligands qui peuvent être identiques ou différents ;
d vaut 1, 2 ou 3 ; et
W est un ligand anionique non coordonné.

13. Procédé selon la revendication 12, dans lequel M est un métal de transition choisi parmi Ru et Os, de préférence Ru.

14. Procédé selon la revendication 12 ou 13, dans lequel chaque L² est indépendamment choisi parmi - H, -CO, -CN, -P(R')₃, -As(R')₃, -CR', -OR', - O(C=O)R', -NR'₂, halogène (par exemple -CI, -Br, -I) et solvant dans lequel chaque R' est indépendamment choisi parmi alkyle substitué ou non substitué, alcényle substitué ou non substitué, alcynyle substitué ou non substitué, hétéroalkyle substitué ou non substitué, alcoxy substitué ou non substitué, cycloalkyle substitué ou non substitué, hétérocycloalkyle substitué ou non substitué, aryle substitué ou non substitué, et hétéroaryle substitué ou non substitué.

15. Procédé selon l'une quelconque des revendications précédentes, dans lequel le catalyseur à métal de transition est
